# EUROPEAN PATENT SPECIFICATION

(11) **EP 2 512 685 B1**
(45) Date of publication and mention of the grant of the patent: **22.07.2015**
(21) Application number: 10798235.7
(22) Date of filing: 17.12.2010
(51) Int. Cl.: B05B 7/00, B05B 11/04, A61Q 5/10, A61K 8/04

(54) **PERSONAL CARE COMPOSITION FOAMING PRODUCT**
KÖRPERPFLEGENDES SCHAUMFÖRMIGES PRODUKT UND SCHAUMABGABEVORRICHTUNG
PRODUIT MOUSSANT POUR L'HYGIENE CORPORELLE ET DISTRIBUTEUR DE MOUSSE

(30) Priority: 18.12.2009 US 287923 P
(43) Date of publication of application: 24.10.2012
(73) Proprietor: The Procter & Gamble Company, Cincinnati, OH 45202 (US)
(72) Inventor: GALAZKA, Sebastian, Karol, Loveland Ohio 45140 (US); LANE, Brandon, Scott, Hamilton Ohio 45013 (US); VOHRA, Firoj, Deerfield Township Ohio 45040 (US); AGOSTINO, Elizabeth, H., Loveland Ohio 45140 (US); KERR, George, Scott, Mason Ohio 45040 (US); LEWIS, Robert, Drennan, West Chester Ohio 45069 (US); LUND, Mark, Thomas, Mason Ohio 45040 (US); DONNER, Christopher, Gerald, Liberty Township, Ohio 45011 (US)
(74) Representative: Boubel, Thomas
(86) International application number: PCT/US2010/061046
(87) International publication number: WO 2011/075652

(56) References cited:
- EP-A1- 2 082 809
- WO-A1-2009/130461
- US-A1- 2005 226 824

## Description

### FIELD OF THE INVENTION

The present invention relates to a higher viscosity personal care composition for use in combination with a foaming dispenser such that a desired foam profile is produced.

### BACKGROUND OF THE INVENTION

Non-aerosol products have become popular, especially for hand soap products. However, designing an acceptable foamed product must focus on achieving foam characteristics desired by consumers for the consumer product.

Desired foam characteristics for consumer products, such as personal care compositions, are dependent upon the end use and consumer expectations. If a consumer foamed product is expected to be applied to a surface other than the hands of the consumer, there are properties that define acceptable foam characteristics to consumers. Acceptable foam characteristics in personal care composition are exemplified by foam that holds its shape and stays in a consistent form, without any container, in order to transfer from a consumer's hand to the desired location on the consumer's body (e.g., hair, face, arms, legs, etc.). If foam collapses prematurely and becomes a liquid, movement of the consumer's hand, specifically the palm of the hand, from a horizontal position to a non-horizontal position for application causes the foam to run, drip or otherwise move before the foam can be applied to the desired location. This foam characteristic is especially undesirable when working with personal care compositions that can change the color or tint the surface it touches, such as hair dying composition or skin tinting compositions.

Manually-actuable, non-aerosol dispenser useful herein includes both squeeze and pump foamers. The basic mechanism to that liquid from a container reservoir is pumped into a liquid/air mixing chamber where air is mixed with liquid held within the container. Foam is generated (air into liquid) and then passed through one or more screens to refine the foam which is then dispensed. Traditionally, liquids that were used in such foamers were described as having a "high" viscosity when the viscosity (at 25°C) is 100 cps (0.1 pascal seconds) or less. See US 2004/0254253 A1. Although viscosities (at 25°C) of up to 300 cps (0.3 pascal seconds) of liquids for such foamers has been discussed. See US 2004/0213752 A1. Use of polymers to increase the viscosity of a personal cleaning composition that is foamed is known, but with target viscosities of the composition are targeted to be less than 100 cps (0.1 pascal seconds). See WO 91/14759.

The manner under which the manually-actuable, non-aerosol dispenser foamers operate to generate foam, higher viscosity materials (higher than 300 cps or 0.3 pascal seconds) either make squeezing or pumping too difficult as large amounts of work (an applied force moving an object a distance) is required to dispense higher viscosity liquids from such foaming containers. Additionally, foam quality of a higher viscosity liquid can be affected when the liquid is shaken as air is trapped in the higher viscosity liquid - thus the amount of air introduced into the higher viscosity compositions (above 300 cps or 0.3 pascal seconds) is insufficient to produce a desire foam specific volume.

Additional benefits of the present invention include a desired foam specific volume of the resulting foamed personal care composition, reduction in force required to actuate the manually-actuable, non-aerosol dispenser, improved end results of the personal care composition such as improved color results and improved amount of personal care product delivered per stroke.

WO2009130461A1 relates to a pump dispenser for mounting to a liquid container comprises a cylindrical type pump arrangement of the type having a mounting member for mounting the dispenser to the container, a generally cylindrical body located within the mounting member for projection into the container, a turret collar for positioning the body within the mounting member, an elongate piston movably mounted within the body, a seal member operative between the piston and an inner surface of the body to define a liquid pump chamber within the body and an actuator mounted to, or operatively connected with, the piston. The dispenser is adapted to provide an air pump chamber at least partially within the actuator and an outlet passageway arrangement of the dispenser includes at least one constricted orifice through which liquid from the liquid pump chamber is passed.

### SUMMARY OF THE INVENTION

The present application relates to a personal care product comprising a manually-actuable, non-aerosol dispenser 25 equipped with a reservoir 3 comprising a reservoir volume, a mixing chamber 5 and a dispensing head, wherein the reservoir 3 contains a liquid hair coloring mixture comprising:
(i) an oxidative dye precursor,
(ii) a foaming agent,
(iii) an alkalizing agent
(iv) a thickening agent; and
(v) an oxidizing agent;
wherein the volume of the hair coloring mixture is less than the reservoir volume 27; wherein the mixed viscosity of the hair coloring mixture is about 0.4 Pascal seconds (400 cps) to about 1.5 Pascal seconds (1500 cps); wherein when the manually-actuable, non-aerosol dispenser 25 is actuated the hair coloring mixture is mixed with air in an liquid to air ratio of from about 1:6 to about 1:15, preferably from 1:8 to 1:12, more preferably from 1:12 to 1:10, and is dispensed as a foam from the manually-actuable, non-aerosol dispenser 25;
wherein the reservoir 3 is fluidly connected to the mixing chamber 5, the mixing chamber 5 is fluidly connected to the dispensing head; wherein when the manually-actuable, nonaerosol dispenser 25 is actuated, a hair colorant product is dispensed as a foam;
wherein the mixing chamber 5 comprises at least one liquid ingress orifice 11, at least one air ingress orifice 9 and at least one mixing chamber egress orifice 13:
wherein the total area of the at least one air ingress orifice 9 is from about 0.62mm² to about 3.14mm², preferably from about 1.26mm² to about 1.88mm²;
wherein the total area of the at least one liquid ingress orifice is from about 1.5mm² to about 3mm², preferably from about 1.8mm² to about 2.3mm².

### BRIEF DESCRIPTION OF THE DRAWINGS

Figure 1 illustrates an embodiment of the manually-actuable, non-aerosol dispenser cross sectional view;
Figure 1A is a magnified view, taken along lines 1A-1A of Figure 1, of a mesh disposed near a diffuser opening or mixing chamber egress orifice of the dispenser;
Figure 1B is a magnified view, taken along lines 1B-1B of Figure 1, of a mesh, disposed near a dispenser head orifice;

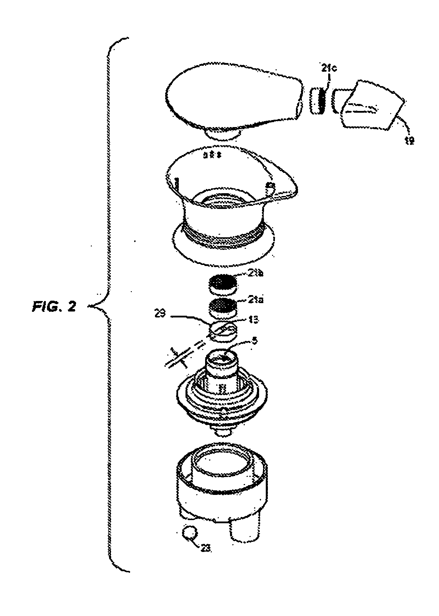

Figure 2 is an exploded view of a dispenser head of the dispenser of Figure 1;
Figure 3 is a cross-sectional view of an alternate embodiment of the manually-actuable, non-aerosol dispenser of the present disclosure;
Figure 3A is a magnified view, taken along lines 3A-3A of Figure 3, of a mesh disposed near a diffuser opening or mixing chamber egress orifice of the dispenser;
Figure 3B is a magnified view, taken along lines 3B-3B of Figure 3, of a mesh, disposed near a dispenser head orifice; and

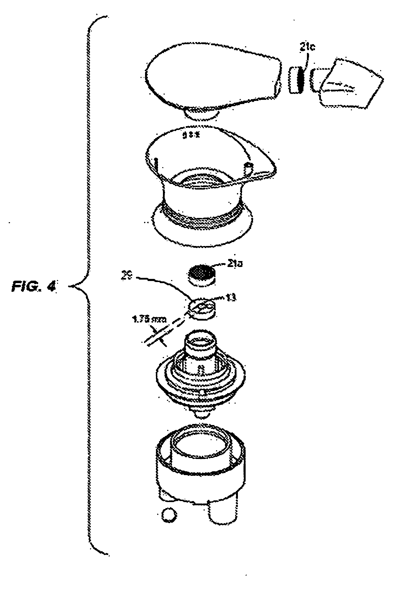

Figure 4 is an exploded view of a dispenser head of the dispenser of Figure 3.
Figure 5 is a perspective view of the mixing device described for the viscosity test method below.
Figure 6 is a front view of the mixing device described for the viscosity test method below.
Figure 7 is a back view of the mixing device described for the viscosity test method below.

### DETAILED DESCRIPTION OF THE INVENTION

The personal care composition of the present application comprises certain rheological characteristics, such as having a desired mixed viscosity or low-shear viscosity and that the composition is shear-thinning (e.g. comprising a viscosity which decreases as shear is applied to the composition) during dispensing of the personal care composition. Lastly, the personal care composition should, after dispensing as a foam and subsequent foam collapse, have a low-shear viscosity which is higher than the mixed viscosity.

The desired viscosity profile ensures a desired consumer experience when interacting with the personal care composition. The foam specific volume is impacted by the rheological properties and the liquid to air ratio. The rheology properties of the composition after it is foamed, and the foam collapsed, is selected such that the composition does not drip or run from the surface on which it is applied, such as hair.

As used herein "mixed viscosity" is the viscosity of the personal care composition prepared and measured by the methods defined below. In one embodiment, a hair colorant composition where two or more sub-components are mixed together, such as a sub-component comprising an oxidative dye precursor (tint composition) and a sub-component comprising an oxidizing agent (developer composition), by a consumer just prior to use of the composition, the mixed viscosity would be the viscosity of the resulting mixture of the tint composition and developer composition rather than the viscosity of the individual sub-components prior to mixing together.

The mixed viscosity of the personal care composition is from about 400 cps (0.4 pascal seconds) to about 1500 cps (1.5 pascal seconds), preferably from about 450 cps (0.45 pascal seconds) to about 1000 cps (1.0 pascal seconds). The personal care composition may comprise components that will affect the mixed viscosity, such a dyes, alkali component content or salt content. A suitable thickener should be selected to adjust the mixed viscosity of the personal care composition into the desired range.

For example, a personal care composition which is a hair colorant formulation comprising a high total dye content and a low alkali content represent dark shades, such a black hair colors, may have a mixed viscosity from about 500 cps (0.5 pascal seconds) to about 1500 cps (1.5 pascal seconds).

For example, a personal care composition which is a hair colorant formulation comprising dyes relating to red shades, such as red or auburn hair colors, may have a mixed viscosity from about 400 cps (0.4 pascal seconds) to about 800 cps (0.8 pascal seconds).

For example, a personal care composition which is a hair colorant formulation comprising low total dye content and high alkali content representing light shades, such as blond colors, may have a mixed viscosity of from about 400 cps (0.4 pascal seconds) to about 700 cps (0.7 pascal seconds), such as 450 cps (0.45 pascal seconds) to about 700 cps (0.7 pascal seconds).

If the personal care product is not a product that requires mixing before use, such as shampoos, conditioners, lotion, cosmetics (e.g., foundation, blush, eye shadow), perfume, facial cleansers and the like, then the composition viscosity is from about 400 cps (0.4 pascal seconds) to about 1500 cps (1.5 pascal seconds), preferably from about 450 cps (0.45 pascal seconds) to about 1000 cps (1.0 pascal seconds). As used herein, the "composition viscosity" means the composition as it exists within the reservoir of the manually-actuable, non-aerosol dispenser when a consumer would be dispensing the personal care product.

The foamed personal care composition, after the foam has collapsed, preferably has a low-shear viscosity more than 1000 cps (1.0 pascal seconds), more preferably more than 10000 cps (10.0 pascal seconds), preferably from 50000 cps (50.0 pascal seconds) to 150000 cps (150 pascal seconds), preferably from 60000 cps (60.0 pascal seconds) to 140000 cps (140.0 pascal seconds). As used herein "low-shear viscosity" means the viscosity of the personal care composition measured according the methodology discussed below and recorded at a shear rate of 0.01 s⁻¹. The low-shear viscosity is believed to represent the viscosity of the personal care composition after the personal care composition is foamed and then collapses.

The personal care composition may comprise components that will affect the low-shear viscosity, such a dyes, ammonia content or salt content.

For example, a personal care composition which is a hair colorant formulation comprising a high total dye content and a low ammonia content represent dark shades, such a black hair colors, may have a low-shear viscosity from about 80000 cps (80.0 pascal seconds) to about 120000 cps (120.0 pascal seconds).

For example, a personal care composition which is a hair colorant formulation comprising dyes relating to red shades, such as red or auburn hair colors, may have a low-shear viscosity from about 100000 cps (100.0 pascal seconds) to about 150000 cps (150.0 pascal seconds).

For example, a personal care composition which is a hair colorant formulation comprising low total dye content and high ammonia content representing light shades, such as blond colors, may have a low-shear viscosity of from about 50000 cps (50.0 pascal seconds) to about 90000 cps (90.0 pascal seconds).

### Foam

As used herein "foam" means a personal care composition which after being passed through a manually-actuable, non-aerosol dispenser has bubbles that sustain their shape and give a volume independent of any type of container. The foam preferably comprises a uniform bubble size. It is preferably that the volume is a foam specific volume from about 6 ml/g to about 14 ml/g, such as about 7.5 ml/g to about 12 ml/g, such as from about 8 ml/g to about 11 ml/g immediately after dispensing. One embodiment is a foam specific volume of about 10 ml/g immediately after dispensing.

As used herein "stroke" means deflecting a reservoir that is placed against a vertical flat surface, such as a wall, on the side of the reservoir opposite the wall, 30 mm towards the wall at a rate of 20 mm per second. "Squeeze" or "dispensed" are also included in the term "stroke".

A manually-actuable, non-aerosol dispenser is optionally designed to have a foam output per stroke or squeeze from about 0.5 gram/stroke to about 5.0 gram/stroke, preferably about 0.8 gram/stroke to about 4.0 gram/stroke, preferably from about 1.0 gram/stroke to about 4.0 gram/stroke. In one embodiment, the manually-actuable, non-aerosol dispenser is optionally designed to have a foam output per stroke or squeeze from about 1.8 gram/stroke to about 2.2 gram/stroke.

A manually-actuable, non-aerosol dispenser is optionally designed to have a foam output per stroke or squeeze from about 3 ml/stroke to about 70 ml/stoke, preferably from about 76 ml/stroke to about 48 ml/stroke, preferably from about 8 ml/stroke to about 44 ml/stroke, preferably from about 18 ml/stroke to about 22 ml/stroke.

The minimum time for the foam to retain its volume is at least long enough to transfer from a user's hand to the desired location, such as on the hair, e.g. the foam substantially maintains its shape and foam specific volume. A preferred minimum time is for at least 10 seconds, for example at least 12, or at least 15 seconds. It could be longer minimum time if a quantity of foam, e.g. a bowl full by a hair dresser, is generated and spreading on the head only starts once the bowl full is readily made, such as 5 minutes or such as 10 minutes.

In order to fulfill the coloring action, oxidative hair colorant compositions need to reach and disperse on the hair. Hence a foam oxidative hair color composition needs to collapse within the time usually allocated for hair coloring (e.g., 10 to 30 minutes). The collapse of the foam could be as quickly as 3 to 10 minutes but may be up to 15 minutes, or up to 30 minutes.

The amount of sebum on hair can affect the foam and cause it to collapse. The more sebum on the hair, the faster the foam collapses on the hair.

### Thickening agents

Suitable for use in the personal care compositions to achieve the desired mixed viscosity (as defined herein) and low-shear viscosity (as defined herein) include salts of fatty acids represented by the formula:

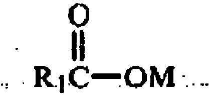

Wherein R₁ is a hydrocarbon radical of the formula CₙH₂ₙ₋₁, CₙH₂ₙ₋₁ or CₙH₂ₙ₋₃ and n is an integer from 12 to 24. M is selected as hydrogen, sodium, potassium or ammonium. Examples of suitable fatty acid salts include ammonium stearate, potassium stearate, ammonium oleate and ammonium ricinoleate. Ammonium salts may result from *in-situ* formation from fatty acids and ammonia, such as oleic acid and ammonia.

The salts of fatty acids may be present in the tint composition from about 0.05% to about 10%, such as about 0.1% to about 8%, such as from about 1% to about 5% by weight of the tint composition.

Polysaccharides and copolymers of polysaccharides and synthetic monomers are also useful in the present invention. Such saccharides useful herein include: glucose, galactose, mannose, arabinose, xylose, fucose, fructose, glucosamine, galactosamine, glucuronic acid, galacturonic acid, and 5 or 6 membered ring polyalcohols. Also included are hydroxymethyl, hydroxyethyl and hydroxypropyl derivatives of the above saccharides. Xanthan gum, e.g., CP Kelco's KELTROL® T, (molecular weight about 2,000,000) is also a suitable polymer. Also suitable include gellan gum (e.g., CP Keloc's KELCOGEL® AFT), pectine, alginate, arabinogalctan, carageenan, rhamsan gum and furcellaran gum. Suitable for use as a thickener herein include cellulose derivatives such as hydroxyethyl- and carboxymethylcellulose and guar gums such as hydroxypropyltrimethyl ammonium guar gum. Specific examples include: a nonionic hydroxyethyl cellulose polymers (e.g., Aqualon/Hercules Incorporated's NATROSOL® 250MXR and NATROSOL® 250HR); and cationic hydroxyethyl cellulose polymers (e.g., Union Carbide Corporation's JR-400 and LM-200).

Some additional polymers suitable for use as a thickener include polyvinyl pyrrolidone and copolymers of vinylpyrrolidone such as those containing vinyl acetate, dimethylaminoethylmethacrylate and quaternary versions of the same with methyl sulfates, and polymers and copolymers of vinyl alcohol and vinyl acetate. Some acrylic polymers suitable for use herein include polyacrylic acid (e.g., Noveon's CARBOPOL® polymers), polyacrylamide, copolymers with esters of acrylic acid and methacrylic acid and copolymers of methylvinylether and maleic anhydride.

Further suitable thickeners are listed in the Glossary and Chapters 3 (alkyl and hydroxyalkylakylcellulose), 4 (carboxymethylcellulose), 12 (hydroxyethylcellulose) and 13 (hydroxypropylcelluose) of the Handbook of Water-Soluble Gums and Resins. Robert L. Davidson, McGraw-Hill Book Co., New York, N. Y., 1980.

The amount of polymeric thickener found useful in the present compositions is about 0.1% to about 20%, preferably from about 0.1% to about 10% by weight of the personal care composition or a portion thereof, such as an oxidative tint component.

### Solvents

If necessary, solvents such as water, lower aliphatic alcohols, for example aliphatic alcohols with from 1 to 4 carbon atoms such as ethanol, propanol and isopropanol, or glycols such as glycerin and 1,2-propylene glycol may be utilized for the personal care composition, such as the hair coloring mixture, in concentrations of from 0.1 to 30 percent by weight.

### Perfume

The personal care compositions may comprise a perfume component or components. Perfumes are often a mixture of components such as essential oils, aroma compounds and solvents (ethanol, water and perfume oils, such as jojoba oil) to provide a sensorial experience with top, middle and base notes selected for the personal care composition. Such components may be found in "Perfume and Flavor Chemicals (Aroma Chemicals)," Steffen Arctander, published by the author, 1969.

### Foaming agent

The foaming agent may be anything so long as the foaming agent has foaming properties including surfactants such as nonionic, anionic, cationic and amphoteric surfactants. Preferred foaming agents include amphoteric surfactants. Useful surfactants are discussed in US 20040213752 in paragraphs [0024] - [0027].

Useful anionic surfactants include alkyl ether carboxylates, alkyl ether sulphates, alkyl glyceryl sulphonates, alkylamido ether sulphates, alkylarylpolyether sulphates, alkyl monoglyceride sulphates, alkyl ether sulphonates, alkylamide sulphonates; alkyl succinates, alkyl sulphosuccinates, alkyl ether sulphosuccinates, alkylamide sulphosuccinates; alkyl sulphosuccinamates; alkyl sulphoacetates; alkyl ether phosphates; acyl sarcosinates, N-acyl methylaminopropionate; acyl isethionates, N-acyltaurates; acyl lactylates; carboxyalkyl ether of alkyl polyglucosides; alkyl lecithin derivatives. In one embodiment, an anionic surfactant is selected as C8-C30 alkyl ether phosphates having from 1 to 20, preferably 2 to 10 ethylene oxide units, and a non-ionic surfactant selected from polyoxyethylene alkyl ethers having at least 25, preferably from 100 to 200 ethylene oxide units.

Useful nonionic surfactants includes one or more polyethyleneoxide chains include the following compounds: polyoxyethylene alkyl ethers, polyethyleneglycol fatty acid esters, polyoxyethylene castor oil, polyoxyethylene hydrogenated castor oil, polyoxyethylene fatty amides and their momoethanolamine and diethanolamine derivatives and polyethoxylated fatty amines. In one embodiment include polyoxyethylene alkyl ethers or polyethylene glycol fatty acid esters having at least about 25, preferably from about 50 to 200, most preferably from about 100 to 200 ethylene oxide units, for example ceteareth-25, steareth-100, steareth-150 and steareth-200.

The amount of foaming agent found useful in the present compositions is about 0.1% to about 20%, preferably from about 0.1% to about 10% by weight of the oxidative tint component.

### Manually-Actuable, Non-Aerosol Dispensers

Manually-actuable, non-aerosol dispensers for foam generation are known in the art. These foam dispensers comprise a reservoir for holding a liquid to be dispensed in the form of foam with an assembly which can be mounted on or in an opening of the reservoir. The assembly comprises a dip tube which extends into the reservoir and into contact with the personal care composition, then the dip tube extends in the opposite direction from the reservoir into a mixing chamber, a liquid pump for pumping the liquid from the reservoir and an air pump to mix air with the liquid in the mixing chamber in order to form foam. The foam is dispensed out of the mixing chamber and through a dispensing channel out of a dispensing head comprising a dispensing orifice. In the dispensing channel one or more porous elements such as sieves or screens that may be arranged to form homogeneous foam.

The amount of work required for dispensing the personal care composition with the higher viscosities described herein (higher than a mixed viscosity of 300 cps (0.3 pascal seconds)) is unique verses known lower viscosity personal care compositions. It is unique in that with lower viscosity personal care composition, more work is expended moving air than the liquid in such systems. For higher mixed viscosity personal care compositions (higher than 300 cps (0.3 pascal seconds)), more work is expended to move the liquid than the air in such systems.

The use of higher viscosity personal care compositions and the amount of work required to move the higher viscosity personal care composition further poses unique problems relating the amount of shear generated in the manually-actuable, non-aerosol dispensers suitable for use herein. The use of higher viscosity personal care compositions further affects the ratio of liquid to air. The amount of work, shear generation, residence time in the shear and liquid to air ratio are aspects that can be attributed to the manually-actuable, non-aerosol dispenser structure.

The ratio of liquid to air is from about 1:6 to about 1:15, preferably from about 1:8 to about 1:12, preferably 1:10.

Applicants have found that this range of foam specific volume gives a desired experience by consumers, with the foamed personal care composition being neither too wet (resulting in running or dripping) or too dry (low amounts of product deposited). The foam specific volume will somewhat be controlled by the choice of manually-actuable, non-aerosol dispenser (discussed further below). Pump foamers often have a narrower range of foam specific volume whereas squeeze foamers have a broader range of foam specific volume as the user of the squeeze foamer may vary the amount of work applied from squeeze to squeeze by the consumer.

Suitable manually-actuable, non-aerosol dispenser structure include the dimensions of the dip tube, dimensions of the air ingress into the mixing chamber, mixing chamber dimensions, including the ingress and egress orifices from the mixing chamber, dispensing channel dimensions, porous elements (such as screens or meshes) and dispensing head orifice.

The manually-actuable, non-aerosol dispenser may be a pump or squeeze foamers. Suitable examples of pump foamers are exemplified in EP 0613728 B1, WO 97/013585 A1 and EP 1716933 A1. Suitable squeeze foamers are exemplified by the following patents: US 3,709,437; US 3,937,364; US 4,022,351; US 4,147,306; US 4,184,615; US 4,598,862; and US 4,615,467; and FR 2,604,622. One particular example of a squeeze foamer useful herein is a squeeze foamer that is able to dispense from an upright or inverted position such as the one discussed in US 6,604,693 assigned to Taplast, and more specifically, at column 2, line 65, through column 4, line 67 of that patent.

The manually-actuable, non-aerosol dispenser comprises a reservoir. The reservoir comprises a volume such that the reservoir volume is larger that the volume of the personal care composition contained within the reservoir. The area of the reservoir that is not occupied by the personal care composition is the head space. The head space should remain relatively free of the personal care composition or bubbles of the personal care composition. If the reservoir is shaken or inverted while the personal care composition is contained therein, the head space should remain relatively free of the personal care composition or bubbles thereof. As used in this paragraph, "relatively free" means less than 50%, such as less than 75%, such as less than 90%, such as 75% to 100% of the head space volume is free from the personal care composition or bubbles thereof.

The reservoir is selected to have enough volume to contain the personal care composition, any mechanism for foaming the personal care composition and head space. The reservoir volume in one embodiment is selected to be from about 100 mL to about 500 mL, from about 150 mL to about 400 mL, such as 250 mL. The ratio of the reservoir volume to personal care composition volume is from about 0.30 to about 0.70, such as from about 0.40 to about 0.55.

If the manually-actuable, non-aerosol dispenser is selected as a squeeze foamer, the shape of the reservoir may be selected such that when the personal care composition is contained within the reservoir, the force required per volume displacement may be optimized. In one embodiment, the force required per volume displacement is optimized when the shape of the bottle is selected to have an elliptical cross-section as viewed from vertical axis of the bottle (from the top or bottom of the bottle). The elliptical cross-section is preferably concentric such that a neck suitable for a threaded or snap-on cap may be used to close the reservoir. The major axis of the elliptical cross-section is orientated such that it is perpendicular to the force applied to the reservoir surface

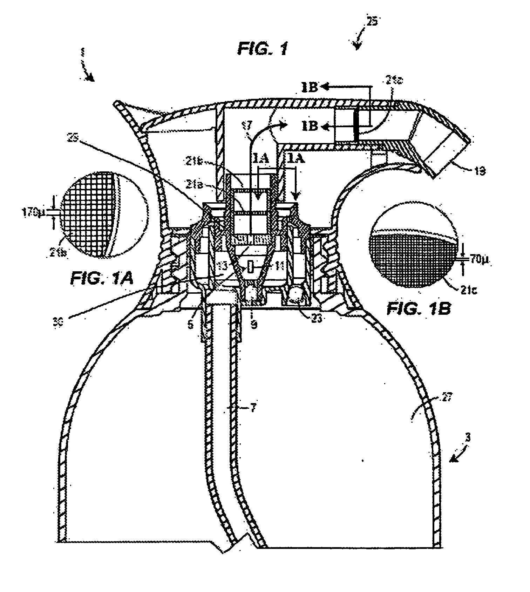

Figure 1 illustrates a general structure for a personal care composition product (25) comprising a foamer assembly (1) and a reservoir (3).

The reservoir (3) having a reservoir volume (27) that contains the personal care composition is fluidly connected to the mixing chamber (5) such that the personal care composition is transported from the reservoir (3) when the manually-actuable, non-aerosol dispenser (25) is dispensed (e.g., "stroke"). The fluid connection is a dip tube (7). The dip tube (7) diameter for the present personal care composition having a relatively higher viscosity requires a relatively larger diameter in order to allow for easy dispensing (low amount of force needed to dispense) and to achieve the desired foam specific volume.

The dip tube (7) diameter is preferably selected to have a diameter of greater than 2.0 mm, preferably from about 2.0 mm to about 5.0 mm, more preferably from about 2.5 mm to about 4.0 mm. The viscosity of the liquid with a dip tube (7) diameter between about 2.0 mm and about 4.0 mm allows for the liquid to be conveyed from the reservoir (3) into the mixing chamber (5) with lower amounts of force by the user during dispensing (e.g., "stroke") while achieving the desired foam density discussed herein.

The mixing chamber (5) comprises at least one air ingress orifice (9), at least one liquid ingress orifice (11) and at least one mixing chamber egress orifice (13). The mixing chamber (5) further comprises an internal volume and an exterior wall, which defines the internal volume of the mixing chamber (5). The mixing chamber (5) allows for the combination of the personal care composition and air to begin the formation of the foamed personal care composition. Modification of the various orifice (9, 11, 13) areas (the two-dimensions of the indicating orifices that comprise part of the mixing chamber (5) exterior wall) can affect the foam specific density, particularly the correlation of the air ingress orifice (9) and the liquid ingress orifice (11) such that the liquid to air ratio is appropriate.

The air ingress orifice (9) is suitable to convey air that has entered into the headspace of the reservoir (3). The mixing chamber (5) may comprise more than one air ingress orifice (9). In one embodiment, the mixing chamber (5) comprises one air ingress orifice (9). The area of the air ingress orifice (9) may be from about 0.62 mm² (about a 0.2 mm diameter circular air ingress orifice) to about 3.14 mm² (about a 1 mm diameter circular air ingress orifice), preferably from about 1.26 mm² (about a 0.4 mm diameter circular air ingress orifice) to about 1.88 mm² (about a 0.8 mm diameter circular air ingress orifice). If more than one air ingress orifice (9) is selected, the total area of all air ingress orifices (9) should be used. Communication of the air in to the mixing chamber (5) via the air ingress orifice (9) can be and indirect communication with the mixing chamber (5) or a direct communication with the mixing chamber (5).

Similarly, the liquid ingress orifice (11) is suitable to fluidly convey the personal care composition into the mixing chamber (5) from the reservoir (3), preferably via a dip tube (7). In one embodiment, the mixing chamber (5) comprises more than one liquid ingress orifice (11). In one embodiment, the mixing chamber (5) comprises three liquid ingress orifices (11). The area of the liquid ingress orifice (11) should be from about 1.5 mm² to about 3 mm². In one embodiment the liquid ingress orifice (11) should be from about 1.8 mm² to about 2.3 mm². If more than one liquid ingress orifice (9) is selected, the total area of all air ingress orifices (9) should be used. For example, a total area of 2.0 mm² for three liquid ingress orifices (11) would equate the total areas of all three liquid ingress orifices (11) combined. The fluid conveyance from the reservoir (3) to the mixing chamber (5) may be an indirect communication pathway with the mixing chamber (5) or a direct communication pathway with the mixing chamber (5).

As used herein "indirect communication" means that the conveyance of the air or personal care composition to the mixing chamber (5) travels along a pathway through some other physical structure before entering into the mixing chamber (5). For example, the air or personal care composition will come into contact with the exterior wall of the mixing chamber (5) before entering into the mixing chamber (5) through the respective orifice (9, 11). In one embodiment, a void volume (30) is contiguous with the exterior wall of the mixing chamber (5). The air or the personal care composition is conveyed from the reservoir, through the dip tube (7) into the void volume (30) external to the mixing chamber (5). The void volume (30) is in air and/or in liquid communication with the air ingress orifice (9) and/or the liquid ingress orifice (11), respectively.

As used herein "direct communication" means that the conveyance of the air or personal care composition to the mixing chamber (5) travels directly into the mixing chamber (5). For example, the air or personal care composition will come into contact with the internal volume of the mixing chamber (5) through the respective orifice (9, 11) without contacting a component exterior to the mixing chamber (5).

In one embodiment, the mixing chamber egress orifice (13) is selected to create an increase in pressure within the mixing chamber (5). The mixing chamber (5) may comprise more than one mixing chamber orifice (13). In one embodiment, the mixing chamber (5) comprises one mixing chamber egress orifice (13).

The mixing chamber (5) has an outer wall creating an internal volume of the mixing chamber (5). The top edge of the outer wall defines a circumference. The circumference of the top edge will define a top area of the mixing chamber (5). The mixing chamber egress orifice (13) may be the same size area of the top area of the mixing chamber (5), but preferably is selected to be smaller area than the area of the top area of the mixing chamber (5) so as to create an increase in pressure in the mixing chamber (5). The area of the mixing chamber egress orifice (13) may be between about 0.314 mm² (0.1 mm diameter circular orifice) to about 9.42 mm² (3 mm diameter circular orifice). In one embodiment, the mixing chamber egress orifice (13) comprises an area of about 2.512 mm² (0.8 mm diameter circular orifice) to about 5.652 mm² (1.8 mm diameter circular orifice). If more than one mixing chamber egress orifice (13) is present, the total area of all of the mixing chamber egress orifices should be considered.

In an embodiment, a diffuser plate (29) comprises the mixing chamber egress orifice (13). The diffuser plate (29) may be part of the mixing chamber (5) structure or it may be a separate component that fits into the mixing chamber (5). It is believed that the diffuser plate (29) helps increase the residence time in the mixing chamber (5) and subjects the personal care composition to more time in the shear generated in the mixing chamber (5).

The mixing chamber (5) is fluidly connected to the foamer assembly (1). The personal care composition enters into the mixing chamber (5) via the liquid ingress orifice (11) and mixes with air which enters the mixing chamber (5) via the air ingress orifice (9).

The air is ordinarily supplied from the environment exterior to the manually-actuable, non-aerosol dispenser (25), the air entering the manually-actuable, non-aerosol dispenser (25) after a stroke which is then located in the headspace of the reservoir (3). The controlled entry or exit of air into the manually-actuable, non-aerosol dispenser (25) reservoir (3) headspace may be accomplished by a ball valve (23) or silicone seal or gasket. The ball valve or silicone seal or gasket may be located in the foamer assembly (1) an in communication with the headspace. In one embodiment, the ball valve (23, silicone seal or gasket is located to communicate between the reservoir (3) and the air external to the manually-actuable, non-aerosol dispenser (25) such that when the manually-actuable, non-aerosol dispenser (25) is being dispensed, the ball valve (23) silicone seal or gasket excludes entry of air external to the manually-actuable, non-aerosol dispenser (25) into the reservoir (3) headspace so that the air in the headspace is conveyed to the mixing chamber through the air ingress orifice (9). After dispensing ("stroke"), the ball valve (23), silicone seal or gasket allows entry of air external to manually-actuable, non-aerosol dispenser (25) to enter into the reservoir (3) to refill the headspace for the next stroke.

After the personal care composition and air enter into the mixing chamber (5) and form the foamed personal care composition, the foamed personal care composition exits the mixing chamber (5) via the mixing chamber egress orifice (13), traveling through a foam fluid connection (17) to the foamer assembly (1) and exits the foamer dispensing orifice (19). The foam fluid connection (17) between the mixing chamber egress orifice (13) and the foamer dispensing orifice (19) may have present therein one or more screens or meshes (21a, 21b, 21c) which may be used to modify the foam specific volume. The number of meshes, the size of the openings in the meshes and the frequency of the openings in the meshes may be used to modify the foam specific volume. In one embodiment, at least 2 meshes (21a, 21b) are utilized, wherein the 2 meshes (21a, 21b) are contiguous with each other. The meshes comprise a diameter section and a depth. The diameter section (largest surface area of the mesh) is the portion of the mesh which would be contiguous with another mesh.

At least a lower portion of the dip tube (7) may be angled toward a lowermost front corner of the reservoir (3) when the reservoir (3) is tilted at an angle for optimal squeezing and dispensing of foam, so as to maximize efficient use of the personal care composition in the reservoir (3). The angle of incline of the lowermost portion of the dip tube (7) preferably mimics the angle of incline of the foamer dispensing orifice (19), and both are preferably at an angle downward from a horizontal axis through the mesh closest to the dispensing head orifice (19) in a range of about 30° to about 45°.

In one embodiment, one to three meshes are present in the fluid connection between the mixing chamber egress and the dispensing head orifice. In one embodiment, two meshes (21a, 21b) are located in the foam fluid connection (17) in close proximity to the mixing chamber egress orifice (13), wherein the two meshes (21a, 21b) comprise about 170 micron (µ) opening size and wherein one mesh (21c) is located in close proximity to the foamer dispensing orifice (19), wherein the one mesh (21c) comprises about a 70 micron (µ) opening size.

In one embodiment two meshes (21a, 21b) located in the foam fluid connection (17) in close proximity to the mixing chamber egress orifice (13) and the two meshes (21a, 21b) are contiguous with each other, wherein the two meshes (21a, 21b) comprise about 170 micron (µ) opening size and wherein one mesh (21c) is located in close proximity to the foamer dispensing orifice (19), wherein the one mesh (21c) comprises about a 70 micron (µ) opening size. Each mesh is preferably provided as an injection molded wafer or disc having a cylindrical sidewall and a screen extending across one end of the cylindrical sidewall. The screen does not extend axially (from the top edge of the cylindrical sidewall to the bottom edge of the cylindrical sidewall moving along the y-axis) the entire length of the cylindrical sidewall. As used in this paragraph, "contiguous" means that the two cylindrical sidewalls of the respective wafers or discs are immediately adjacent one another. However, each of the respective wafers is preferably oriented with its screen is facing up, such that even with the two wafers or discs in contact with one another, there is a gap separating the screen of the first disc from the screen of the second disc.

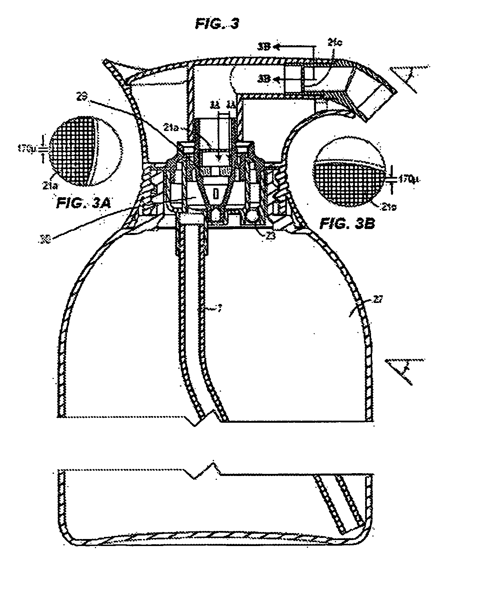

Turning now to Figure 3, a particularly preferred embodiment is illustrated in which only two meshes (21a, 21c) are utilized, one (21a) in close proximity to the mixing chamber egress orifice (13) and the other (21c) disposed close proximity to the foamer dispensing orifice (19).

By varying the size of the mixing chamber egress orifice (13), the number of meshes (21a, 21b, 21c), and the opening size of the screens of the meshes, it is possible to reduce the amount of work required to expel a desired quantity of foam, while substantially preserving the desired foam specific volume. For instance, in an exemplary implementation of the embodiment illustrated in Figure 1, a mixing chamber egress orifice (13) of 1 mm diameter is provided in a diffuser plate (29) [area of orifice is pi * diameter]. In that embodiment, three mesh wafers or discs are provided in the foam fluid connection (17), with each of the first two (21a, 21b) comprising a mesh opening size of about 170 micron (µ), and the third comprising a mesh opening size of about 70 micron (µ).

In an exemplary implementation of the embodiment illustrated in Figure 3, the second mesh (21b) is omitted, the mixing chamber egress orifice is increased to 1.75 mm in a diffuser plate (29) [area of orifice is pi * diameter], the first mesh (21a) has a mesh opening size of about 170 micron (µ), and the mesh wafer or disc (21c) comprises a mesh opening size of about 70 micron (µ) in located in the foam fluid connection (17).

### Personal Care Compositions

### Cationic Deposition Polymers

The personal care composition may comprise a cationic deposition polymer selected from cellulose, guar, cationically modified starch, galactomannan polymer derivative and suitable synthetic deposition polymers.

### Cellulose or Guar Cationic Deposition Polymers

The personal care compositions may include cellulose or guar cationic deposition polymers. Such cellulose or guar deposition polymers have a charge density from about 3 meq/g to about 4.0 meq/g at the pH of intended use of the personal care composition, which pH will generally range from about pH 3 to about pH 9, preferably between about pH 4 and about pH 8. The pH of the compositions are measured neat.

In one embodiment, the cellulose polymers are salts of hydroxyethyl cellulose reacted with trimethyl ammonium substituted epoxide, referred to in the industry (CTFA) as Polyquaternium 10 and available from Amerchol Corp. (Edison, N.J., USA).

Other suitable cationic deposition polymers include cationic guar gum derivatives, such as guar hydroxypropyltrimonium chloride, specific examples of which include the Jaguar series (preferably Jaguar C-17®) commercially available from Rhone-Poulenc Incorporated.

### Cationically Modified Starch Polymer

The personal care compositions may also comprise a water-soluble cationically modified starch polymer. The cationically modified starch polymers have a charge density at least about 3.0 meq/g. The chemical modification to obtain such a charge density includes, but is not limited to, the addition of amino and/or ammonium groups into the starch molecules. Non-limiting examples of these ammonium groups may include substituents such as hydroxypropyl trimmonium chloride, trimethylhydroxypropyl ammonium chloride, dimethylstearylhydroxypropyl ammonium chloride, and dimethyldodecylhydroxypropyl ammonium chloride. See Solarek, D. B., Cationic Starches in Modified Starches: Properties and Uses, Wurzburg, O.B., Ed., CRC Press, Inc., Boca Raton, Florida 1986, pp 113-125.

The source of starch before chemical modification can be chosen from a variety of sources such as tubers, legumes, cereal, and grains. Non-limiting examples of this source starch may include corn starch, wheat starch, rice starch, waxy corn starch, oat starch, cassava starch, waxy barley, waxy rice starch, glutenous rice starch, sweet rice starch, amioca, potato starch, tapioca starch, oat starch, sago starch, sweet rice, or mixtures thereof. Waxy corn starch is preferred.

### Galactomannan Polymer Derivative

The personal care compositions may comprise a galactomannan polymer derivative having a mannose to galactose ratio of greater than 2 : 1 on a monomer to monomer basis, the galactomannan polymer derivative is selected from the group consisting of a cationic galactomannan polymer derivative and an amphoteric galactomannan polymer derivative having a net positive charge.

The gum for use in preparing the non-guar galactomannan polymer derivatives is typically obtained as naturally occurring material such as seeds or beans from plants. Examples of various non-guar galactomannan polymers include but are not limited to Tara gum (3 parts mannose / 1 part galactose), Locust bean or Carob (4 parts mannose / 1 part galactose), and Cassia gum (5 parts mannose / 1 part galactose). Herein, the term "non-guar galactomannan polymer derivatives" refers to cationic polymers which are chemically modified from a non-guar galactomanan polymer. A preferred non-guar galactomannan polymer derivative is cationic cassia, which is sold under the trade name, Cassia EX-906, and is commercially available from Noveon Inc.

The personal care compositions may comprise at least about 0.05% of a galactomannan polymer derivative by weight of the composition. In one embodiment, the personal care compositions comprise from about 0.05% to about 2 %, by weight of the composition, of a galactomannan polymer derivative. Suitable galactomannan polymer derivatives are described in U.S. Patent Publication No. 2006/0099167A1 to Staudigel et al..

### Synthetic Deposition Polymers

Synthetic deposition polymers may also be used in the personal care composition from about 0.05% to about 2% by weight of the personal care composition. Suitable synthetic deposition polymers include those discussed in US 7,585,827, specifically discussed at Col. 5, lines 1-67 and Col. 8, line 5 - Col 15. line 5. The synthetic polymer of 1-Propanaminium, N,N,N-trimethyl-3-[(2-methyl-1-oxo-2-propenyl)amino]-, chloride; (Poly(Methacrylamidopropyl trimethyl ammonium chloride)) is a preferred synthetic polymer.

Another synthetic polymers suitable for the personal care composition include those discussed in US 2004/0010106 and US 2007/0207109. A particularly suitable synthetic polymer is a random copolymer having a net positive charge comprising a nonionic monomer unit of the following formula: 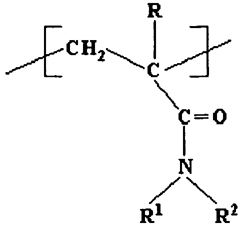 where R is H or C₁₋₄ alkyl; and R¹ and R² are independently selected from the group consisting of H, C₁₋₄ alkyl, CH₂OCH₃, CH₂OCH₂CH(CH₃)₂, and phenyl, or together are C₃₋₆cycloalkyl; and a cationic monomer unit with 2 or more positive charges of the following formula: 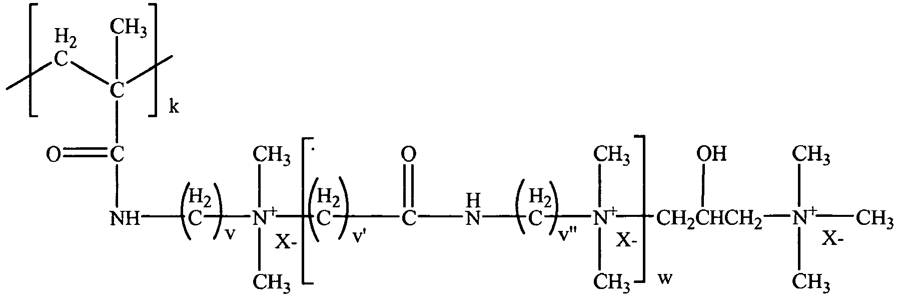 where k = 1, each of v, v', and v" is independently an integer of from 1 to 6, w is zero or an integer of from 1 to 10, and X⁻ is an anion. A preferred cationic monomer is when k=1, v = 3, v' = 1, and v" = 3, w=1, y=1 and X- is Cl- (see paragraph [0051] of US 2007/0207109).

### Exfoliating Agents

The body wash compositions and facial cleanser composition may further comprise particulate cleansing or exfoliating agents. Known particulate cleansing or exfoliating agents are acceptable as long as the particle size is suitable for use with the manually-actuable, non-aerosol dispensers and the components thereof, such as the porous elements.

### Conditioning Agent

The personal care composition may comprise or are used in combination with a conditioning composition comprising a conditioning agent. Conditioning agents suitable are selected from silicone materials, amino silicones, fatty alcohols, polymeric resins, polyol carboxylic acid esters, cationic polymers, cationic surfactants, insoluble oils and oil derived materials and mixtures thereof. Additional materials include glycerin and sorbitol. Particularly useful conditioning materials are cationic polymers. Conditioners of cationic polymer type can be chosen from those comprising units of at least one amine group chosen from primary, secondary, tertiary and quaternary amine groups that may either form part of the main polymer chain, or be borne by a side substituent that is directly attached to the main polymer chain.

Silicones can be selected from polyalkylsiloxane oils, linear polydimethylsiloxane oils containing trimethylsilyl or hydroxydimethylsiloxane endgroups, polymethylphenylsiloxane, polydimethylphenylsiloxane or polydimethyldiphenylsiloxane oils, silicone resins, organofunctional siloxanes having in their general structure one or a number of organofunctional group(s), the same or different, attached directly to the siloxane chain or mixtures thereof. Said organofunctional group(s) are selected from: polyethyleneoxy and/or polypropyleneoxy groups, (per)fluorinated groups, thiol groups, substituted or unsubstituted amino groups, carboxylate groups, hydroxylated groups, alkoxylated groups, quaternium ammonium groups, amphoteric and betaine groups. The silicone can either be used as a neat fluid or in the form of a pre-formed emulsion.

The conditioning agent will generally be used at levels of from about 0.05% to about 20% by weight of the personal care composition, such as from about 0.1% to about 15%, such as of from about 0.2% to about 10%, such as from about 0.2% to about 2% by weight of the personal care composition.

### Hair Colorant

The hair colorant product contains an oxidative tint composition (herein after tint composition) comprising oxidative dye precursors, through which the coloring is produced by the action of oxidizing agents, such as for example hydrogen peroxide, or in the presence of atmospheric oxygen (if necessary with the addition of a suitable enzyme system).

### Oxidative Dye Precursors

The hair colorant compositions may include oxidative dye compounds in the form of primary intermediates or couplers. The compounds suitable for use, in so far as they are bases, may be used as free bases or in the form of their physiologically compatible salts with organic or inorganic acids, such as hydrochloric, hydrobromic, citric, acetic, lactic, succinic, tartaric, or sulfuric acids, or, in so far as they have aromatic hydroxyl groups, in the form of their salts with bases, such as alkali phenolates.

These oxidative dye compounds are well known in the art, and include aromatic diamines, aminophenols, aromatic diols and their derivatives (a representative but not exhaustive list of oxidation dye precursor can be found in Sagarin, "Cosmetic Science and Technology", "Interscience, Special Edn. Vol. 2 pages 308 to 310).

It is to be understood that the precursors detailed below are only by way of example and are not intended to limit the hair care compositions or sub-components such as tint compositions herein. These are: 1,7-Dihydroxynaphthalene (1,7-NAPHTHALENEDIOL); 1,3-Diaminobenzene (m-PHENYLENEDIAMINE); 1-Methyl-2,5-diaminobenzene (TOLUENE-2,5-DIAMINE); 1,4-Diaminobenzene (p-PHENYLENEDIAMINE); 1,3-Dihydroxybenzene (RESORCINOL); 1,3-Dihydroxy-4-chlorobenzene, (4-CHLORORESORCINOL); 1-Hydroxy-2-aminobenzene, (o-AMINOPHENOL); 1-Hydroxy-3-aminobenzene (m-AMINOPHENOL); 1-Hydroxy-4-amino-benzene (p-AMINOPHENOL); 1-Hydroxynaphthalene (1-NAPHTHOL); 1,5-Dihydroxynaphthalene (1,5-NAPHTHALENEDIOL); 2,7-dihydroxynaphthalene (2,7-NAPHTHELENEDIOL); 1,4-Dihydroxybenzene (HYDROQUINONE); 1-Hydroxy-4-methylaminobenzene (p-METHYLAMINOPHENOL); 6-Hydroxybenzo-morpholine (HYDROXYBENZOMORPHOLINE); 1-Methyl-2-hydroxy-4-aminobenzene (4-AMINO-2-HYDROXY-TOLUENE); 1-Methyl-2-hydroxy-4-(2'-hydroxyethyl)aminobenzene (2-METHYL-5-HYDROXY-ETHYLAMINO-PHENOL); 1,2,4-Trihydroxybenzene (1,2,4-TRIHYDROXYBENZENE); 1-Phenol-3-methylpyrazol-5-on (PHENYLMETHYLPYRAZOLONE); 1-(2'-Hydroxyethyloxy)-2,4-diaminobenzene (2,4-DIAMINOPHENOXY-ETHANOL HCL); 1-Hydroxy-3-amino2,4-dichlorobenzene (3-AMINO-2,4-DICHLORO-PHENOL); 1,3-Dihydroxy-2-methylbenzene (2-METHYLRESORCINOL); 1- Amino-4-bis-(2'-hydroxyethyl)aminobenzene (N,N-BIS(2-HYDROXY-ETHYL)-p-PHENYLENE-DIAMINE); 2,4,5,6-Tetraaminopyrimidine (HC Red 16); 1-Hydroxy-3-methyl-4-aminobenzene (4-AMINO-m-CRESOL); 1-Hydroxy-2-amino-5-methylbenzene (6-AMINO-m-CRESOL); 1,3-Bis-(2,4-Diaminophenoxy)propane (1,3-BIS-(2,4-DIAMINO-PHENOXY)-PROPANE);1-(2'-Hydroxyethyl)-2,5-diaminobenzene (HYDROXYETHYL-p-PHENYLENE DIAMINE SULPHATE); 1-Methoxy-2-amino-4-(2'-hydroxyethylamino)benzene, (2-AMINO-4-HYDROXYETHYLAMINOANISOLE); 1-Hydroxy-2-methyl-5-amino-6-chlorobenzene (5-AMINO-6-CHLORO-o-CRESOL); 1-Hydroxy-2-amino-6-methylbenzene (6-AMINO-o-CRESOL); 1-(2'-Hydroxyethyl)-amino-3,4-methylenedioxybenzene (HYDROXYETHYL-3,4-METHYLENEDIOXY-ANILINE HCl); 2,6-Dihydroxy-3,4-dimethylpyridine (2,6-DIHYDROXY-3,4-DIMETHYLPYRIDINE); 3,5-Diamino-2,6-dimethoxypyridine (2,6-DIMETHOXY-3,5-PYRIDINEDIAMINE); 5,6-Dihydroxyindole (5,6-DIHYDROXY-INDOLE); 4-Amino-2-aminomethylphenol (2-AMINOETHYL-p-AMINO-PHENOL HCl); 2,4-Diamino-5-methylphenetol (2,4-DIAMINO-5-METHYL-PHENETOLE HCl); 2,4-Diamino-5-(2'-hydroxyethyloxy)toluene (2,4-DIAMINO-5-METHYLPHENOXYETHANOL HCl); 5-Amino-4-chloro-2-methylphenol (5-AMINO-4-CHLORO-o-CRESOL); 1,3-Bis(N(2-Hydroxyethyl)N(4-amino-phenyl)amino)-2-propanol (HYDROXYPROPYL-BIS-(N-HYDROXY-ETHYL-p-PHENYLENEDIAMINE)HCL); 6-Hydorxyindole (6-HYDROXY-INDOLE); 2,3-Indolinedione (ISATIN); 3-Amino-2-methylamino-6-methoxypyridine (HC BLUE NO. 7); 1-Phenyl-3-methyl-5-pyrazolone (2,4-DIHYDRO-5-METHYL-2-PHENYL-3H-PYRAZOL-3-ONE); 2-Amino-3-hydroxypyridine (2-AMINO-3-HYDROXYPYRIDINE); 5-Amino-salicylic acid; 1 -Methyl-2,6-bis(2-hydroxy-ethylamino)benzene (2,6-HYDROXYETHYLAMINO-TOLUENE); 4-Hydroxy-2,5,6-triaminopyrimidine (2,5,6-TRIAMINO-4-PYRIMIDINOL SULPHATE); 2,2'-[1,2-Ethanediyl-bis-(oxy-2,1-ethanediyloxy)]-bis-benzene-1,4-diamine (PEG-3,2',2'-DI-p-PHENYLENEDIAMINE); 5,6-Dihydroxyindoline (DIHYDROXYINDOLINE); N,N-Dimethyl-3-ureidoaniline (m-DIMETHYL-AMINO-PHENYLUREA); 2,4-Diamino-5-fluortoluenesulfatehydrate (4-FLUORO-6-METHYL-m-PHENYLENEDIAMINE SULPHATE); 1-Acetoxy-2-methylnaphthalene (1-HYDROXYYETHYL-4,5-DIAMINOPYRAZOLE SULPHATE);1-acetoxy-2-methylnaphthalene (2-METHYL-1-NAPHTHOL);2-amino-5-ethylphenol (2-AMINO-5-ETHYLPHENOL);2,4-dichloro-3-aminophenol (3-AMINO-2,4-DICHLOROPHENOL); and p-Anilinoaniline (N-PHENYL-P-PHENYLENEDIAMINE).

The total quantity of the oxidative dye precursors contained in tint composition is up to about 12 percent by weight, especially from about 0.05% to about 6% by weight of the tint composition.

To obtain specific color shades, moreover, additional conventional natural and/or synthetic direct dyes can be contained in the colorant, for example plant pigments such as henna or indigo, triphenylmethane dyes, aromatic nitro dyes, azo dyes, quinone dyes, cationic dyes (Basic dyes) or anionic dyes (Acid dyes).

### Alkalizing Agent

The personal care composition, such as a tint composition for a hair coloring mixture, may comprises an alkalizing agent, preferably a source of ammonium ions and or ammonia. Any agent known in the art may be used such as alkanolamides for example monoethanolamine, diethanolamine, triethanolamine, monopropanolamine, dipropanolamine, tripropanolamine, 2-amino-2-methyl-1,3-propanediol, 2-amino-2-methyl-1-propanol, and 2-amino-2-hydroxymethyl-1,3-propanediol and guanidium salts. Particularly, preferred alkalizing agents are those which provide a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium carbamate, ammonia and mixtures thereof.

The compositions may comprise from about 0.1% to about 10% by weight, such as from about 0.5% to about 5%, such as from about 1% to about 3% of an alkalizing agent, such as a source of ammonium ions.

### Oxidizing Agent

The personal care compositions herein, such as a developer composition for the hair coloring mixture, may comprise at least one source of an oxidizing agent. Preferred oxidizing agents for use herein are water-soluble peroxygen oxidizing agents. "Water-soluble" as defined herein means that in standard condition at least 0.1 g, preferably 1 g, more preferably 10 g of said oxidizing agent can be dissolved in 1 liter of deionized water. The oxidizing agents may be provided in aqueous solution or as a powder which is dissolved prior to use. It should be noted that the inclusion of an oxidizing agent will impact the selection of the thickening agent herein. Not all thickening agents are storage stable in the presence of an oxidizing agent.

The oxidizing is preferably selected from water-soluble oxidizing agents are inorganic peroxygen materials capable of yielding hydrogen peroxide in an aqueous solution, such as hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. Preferred for use in the compositions are hydrogen peroxide, percarbonate, persulphates and combinations thereof.

The oxidative agent may comprise from about 0.1% to about 40% by weight, preferably from about 1% to about 30% by weight, and most preferably from about 2% to about 30% by weight of an oxidizing agent

Another potential oxidizing agent for use herein is a source of peroxymonocarbonate ions. Preferably such a source is formed *in situ* from a source of hydrogen peroxide and a hydrogen carbonate ion source. Such an oxidizing agent has been found to be particularly effective at a pH of up to and including 9.5, preferably 7.5 to 9.5 more preferably about pH 9. Moreover, this system is also particularly effective in combination with a source of ammonia or ammonium ions.

Accordingly, any source of these peroxymonocarbonate ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and as an oxidizing agent. Preferred sources of carbonate ions, carbamate and hydrocarbonate ions are sodium hydrogen carbonate, potassium hydrogen carbonate, ammonium carbamate, and mixtures thereof.

The oxidative agent may comprise from about 0.1% to about 15% by weight, preferably from about 1% to about 10% by weight, and most preferably from about 1% to about 8% by weight of a hydrogencarbonate ion and from about 0.1% to about 10% by weight, preferably from about 1% to about 7% by weight, and most preferably from about 2% to about 5% by weight of the oxidative agent of a source of hydrogen peroxide.

### pH

The compositions may have a pH of from 8 to 12, preferably from 8 to 10. For embodiments comprising a peroxymoncarbonate ion the pH is preferably up to and including pH 9.5, more preferably from about 9.5 to about 7.5, even more preferably from about 9.5 to about 8.4 and most preferably from about 9.4 to about 8.5 and even more preferably about pH 9.3 or 9.0.

If sub-components such as a tint composition or developer composition are utilized, the pH of these sub-components may be different from the personal care composition pH. For example, a tint composition may comprise an alkaline agent which would have an alkaline pH above that described for the personal care composition.

The pH of the compositions can be determined by using either a Mettler Toledo MP220 or a MP225 pH equipment, fitted with a standard laboratory pH electrode. The equipment is calibrated before each use using standard calibration buffers and using the standard calibration procedure.

### Chelants

The hair colorant compositions or sub-components thereof (such as a tint composition or developer composition) comprise a carboxylic acid chelant, a phosphonic acid chelant, a polyphosphoric acid chelant, salts thereof, or mixtures thereof. Suitable chelants include diethylenetriamine pentaacetic acid (DTPA), ethylenediamine-N,N'-disuccinic acid (EDDS), ethylenediamine-N,N'-diglutaric acid (EDDG), 2-hydroxypropylenediamine-N,N'-disuccinic acid (HPDDS), glycinamide-N,N'-disuccinic acid (GADS), ethylenediamine-N-N'-bis(ortho-hydroxyphenyl acetic acid) (EDDHA), diethylene-triamine-penta-(methylenephosphonic acid) (DTPMP), salts thereof, derivatives thereof, or mixtures thereof.

The hair colorant composition or sub-component thereof, such as the tint composition, comprise from about 0.01% to about 5%, from about 0.25% to about 3%, from about 0.5% to about 1% by weight of the hair colorant composition, or sub-component thereof of chelant, salts thereof, derivatives thereof, or mixtures thereof.

### Radical Scavenger

The personal care composition, such as tint compositions for the hair coloring mixture, may further comprise a source of radical scavenger. As used herein the term radical scavenger refers to a species that can react with a carbonate radical to convert the carbonate radical by a series of fast reactions to a less reactive species, i.e. a carbonate radical scavenger.

Suitable radical scavengers for use herein may be selected from the classes of alkanolamines, amino sugars, amino acids, esters of amino acids and mixtures thereof. Particularly preferred compounds are: monoethanolamine, 3-amino-1-propanol, 4-amino-1-butanol, 5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof.

Especially preferred compounds are glycine, sarcosine, lysine, serine, 2 methoxyethylamine, glucosamine, glutamic acid, morpholine, piperidine, ethylamine, 3 amino-1-propanol and mixtures thereof.

The compositions preferably comprise from about 0.1% to about 10% by weight, preferably from about 1% to about 7% by weight of the composition of a radical scavenger.

Preferably, the radical scavenger is present at an amount such that the weight ratio of radical scavenger to carbonate ion is from 2:1 to 1:4. The radical scavenger is also preferably selected such that it is not an identical species as the alkalizing agent. The radical scavenger may be formed *in situ* in the hair coloring mixtures prior to application to the hair fibers.

### Method of Use

### Method of Use

Hair coloring products are usually sold in kits comprising, in individually packaged sub-components in separate containers, such as a tint composition comprising the oxidative dye precursors, alkalizing agent and a thickening agent in a suitable carrier, and a developer composition comprising an oxidizing agent. Generally, the sub-components are provided such that the weight ratio of tint formulation: developer formulation is in the range 5:1 to 1:5, such as 1:1, 1:1.5, 1:2, 1:3 and 1.4 depending on strength of developer composition and tint composition.

The consumer mixes the tint composition and the developer composition together in the reservoir of the manually-actuable, non-aerosol dispenser immediately before use and connects the manually-actuable, non-aerosol dispenser to the reservoir.

The consumer may then shake in a vertically reciprocating motion or in a rotating reciprocating shaking motion for 1 to 15 seconds to mix the tint composition and developer composition without the formation of bubbles in the head space of the reservoir. The consumer then actuates the manually-actuable, non-aerosol dispenser to dispense foam (foamed personal care composition) either into the consumer's gloved hand or directly onto the hair. The foam will collapse to form a liquid hair colorant composition having a low shear viscosity as defined herein. Suitable hair colorant compositions are given in the tables hereinafter.

Generally, a hair coloring method consistent with the foregoing disclosure can include combining an tint composition and a developer composition in a manually-actuable, non-aerosol dispenser to form an hair colorant composition, dispensing the hair colorant composition in the form of a foam, contacting the dispensed foam with hair for a time period sufficient to color the hair and, rinsing the hair contacted with the hair colorant composition.

The dispenser preferably is equipped with a reservoir that includes a reservoir volume, a mixing chamber, a dispensing head, at least one mesh disposed intermediate a mixing chamber egress orifice of the mixing chamber and a dispenser head orifice of the dispensing head. Further, the dispenser includes a dip tube in fluid communication with the mixing chamber and the reservoir volume.

A more specific method or process of coloring hair using the foamers of the present disclosure will now be described. A method of coloring hair with at least 100 grams of hair coloring foam, preferably about 110 g, and more preferably, 120 g, comprises the following steps:
(1) Creating an hair colorant composition by combining a tint composition and a developer composition in a manually-actuable, non-aerosol dispenser equipped with a reservoir comprising a reservoir volume, a mixing chamber, a dispensing head, at least one mesh disposed intermediate a mixing chamber egress orifice of the mixing chamber and a dispenser head orifice of the dispensing head, each of the at least one mesh having a screen opening size in the range of about 70 micron to about 170 micron, and a dip tube in fluid communication with the mixing chamber and the reservoir volume, the reservoir being a squeezable container that, upon application and maintenance of a force from opposing directions, compresses and directs the hair colorant composition within the reservoir into the dip tube.
(2) Mixing the tint composition and the developer composition by shaking the manually-actuable, non-aerosol dispenser. As used herein, shaking includes at least turning the manually-actuable, non-aerosol dispenser a plurality of times back and forth to mix the oxidative hair colorant composition components with one another until they have formed a homogeneous mixture.
(3) Squeezing the exterior of the reservoir of the manually-actuable, non-aerosol dispenser, thereby dispensing the hair colorant composition from the reservoir in the form of a foam, such that the foam is expelled through the dispensing head orifice.
(4) Applying the dispensed foam to hair to be colored.
(5) Repeating steps (2) and (3) a plurality of times, the plurality of times to be no more than 60 times, preferably no more than 50 times, and more preferably, no more than 45 times.
(6) Permitting the hair to react with the dispensed foam for a predetermined time, the predetermined time being commensurate with the time it takes for the hair to reach the color which the hair colorant composition is formulated to achieve, and the predetermined period of time preferably not exceeding 10 minutes.
(7) Rinsing the hair to which the hair colorant composition was applied with water to remove any remaining hair colorant composition.

The method may include an optional additional step (8) of treating the hair and scalp with a post-colorant care composition. When present, the optional conditioning agent can be provided in a third container. In one embodiment, the content of the third container can be applied (after an optional rinse step) as a post-treatment immediately after the oxidative tint composition resulting from the mixture of the other containers.

The methods of coloring hair also comprise embodiments whereby the hair colorant composition is applied to the hair and preferably the mixture is worked for a few minutes (to ensure uniform application to all of the hair). The hair colorant composition is then allowed to remain on the hair in order for the color to develop for a time period of less than about 20 minutes, preferably less than about 15 minutes, more preferably from about 5 minutes to about 10 minutes, most preferably for about 10 minutes. The consumer then rinses his/her hair thoroughly with tap water and allows it to dry and or styles the hair as usual.

The method of coloring the hair is a sequential hair coloring method comprising the steps of at least two sequential hair color treatments wherein the time period between each treatment is from 1 to 60 days, preferably from 1 to 40 days, more preferably from 1 to 28 days, even more preferably from 1 to 14 days and most preferably from 1 to 7 days. In such embodiments the time that the composition is retained on head may be less than about 20 minutes and is preferably less than about 10 minutes and most preferably from about 2 minutes to about 5 minutes.

### Test Methods

### Viscosity

### Sample preparation

The tint composition and developer composition are combined to make an oxidative hair colorant composition. The sample preparation of the oxidative hair colorant composition should be as follows:
1. combine, in a 1:1 weight ratio, the tint composition and the developer composition in a closable container from which it can be dispensed. The container should be closed or capped.
2. the closable container is then placed into a Mechanical Mixer (described below) and is shaken for 15 seconds.
3. The contents of the closed container poured into a 100 tall container available from FlackTek Inc. is then placed onto a DAC 800 FVZ SpeedMixer from FlackTek Inc. set to 1950 rpm for 10 seconds to draw any bubbles in the out of the sample.
4. A watch glass is used to contain the bubbles or foam on the top of the sample, while the liquid is decanted into a container suitable for measuring viscosity.
5. The sample is then measured for viscosity.

### Mechanical Mixer

The Mechanical Mixer (31) is a device to replicate a shaking motion of a consumer. By shaking motion, it is a motion using the elbow as a pivot (fulcrum) point, with the wrist in a straight position and the arm is moved about the pivot point in an up and down motion.

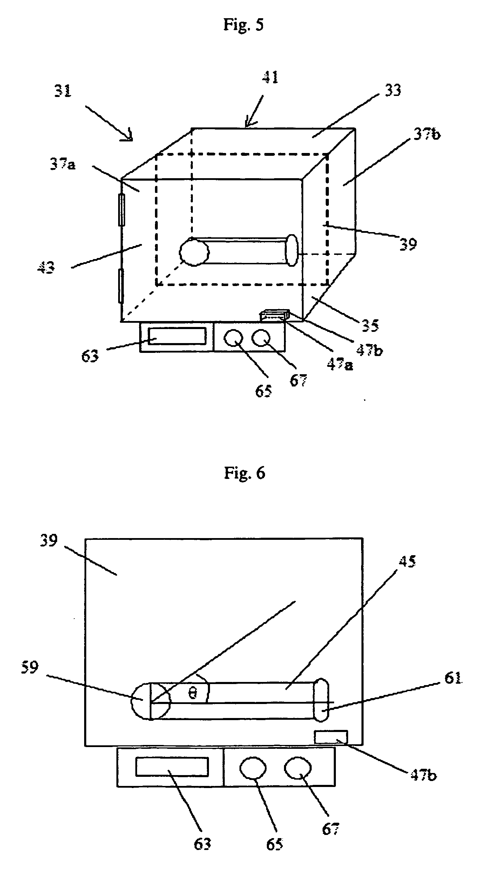

The Mechanical Mixer (31) in Fig. 5 is an enclosed device having a top wall (33), a bottom wall (35), two vertical side walls (37a, 37b), a middle panel (39), a back panel (41) and a hinged door (43) which hingeably opens and shuts to allow access to the enclosed device. A metal bar (45), described further below, and a door safety switch (47) are located on one side of the middle panel (39) between the middle panel (39) and the hinged door (43). A air controlled solenoid motor (49), electrical air dump mechanism (51), air regulator (53), power supply (55) and safety relay (57) are located on a second side of the middle panel (43) between the middle panel (43) and the back panel (41).

The Mechanical Mixer (31) from a view shown in Fig. 6 (which does not shown the hinged door (43), top wall (33), bottom wall (35) or two vertical side walls (37a, 37b)) comprises a 45.16 cm length metal bar (45) having a pivot point (59) on one end of the bar (45) and a clamping means (61) on a second end of the bar (45) that is capable of holding a container of the oxidative hair colorant composition while the Mechanical Mixer (31) is in operation. The metal bar (45) should travel in an upwards and downwards direction through a 44° angle (34.5 cm arc) shown as θ. The pivot point (59) is moved through the desired angle via an air controlled solenoid motor (49) capable of 45 cycles (up and down motion) in 15 seconds.

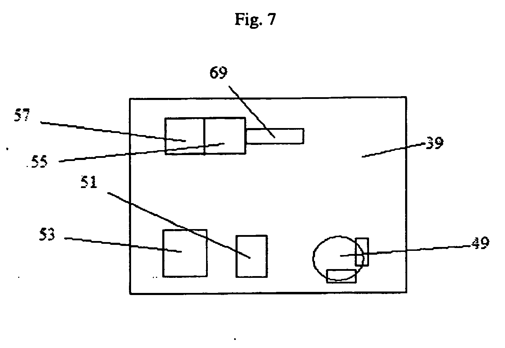

In Fig. 7 (which does not shown the back panel (41), top wall (33), bottom wall (35) or two vertical side walls (37a, 37b)), the air controlled solenoid motor (49) can be see and is connected to an electrical air dump mechanism (51). The air dump mechanism (51) is connected to an air regulator (53), which generates the air pressure to drive the air controlled solenoid motor (49). The air regulator (53) is connected to a power supply (55) and preferably a safety relay (57) as there is a pressurized air system for the Mechanical Mixer (31). The safety relay (57) is connected to a door safety switch (47), comprising two halves (47a, 47b), the first half (47a) is located partially on the hinged door (43) and the second half (47b) is inside the space enclosed by the top wall (33), bottom wall (35), two vertical walls (37a, 37b), the middle panel (39) and the hinged door (43), the two halves (47a, 47b) being located adjacent to each other in order to complete a circuit with the safety relay (57). When the two halves (47a, 47b) of the door safety switch (47) are separated as the hinged door (43) is opened, the circuit with the safety relay (57) is not completed and the Mechanical Mixer is stopped.

It is preferable to have a programmable relay (63), start button (65), stop button (67) located outside of the enclosed device. The programmable relay (63) may be connected to power supply (55) via a terminal strip (69), bus or other similar device. The programmable relay (63) allows for setting of time of operation, modification of angle of movement, speed of movement and the like. The start button (65) and stop button (67) are likewise located outside of the enclosed device, preferably located adjacent to the hinged door (43). If the programmable relay (63) is utilized, the desired settings can be imputed for each sample and the start button (65) and stop button (67) can control the operation of the Mechanical Mixer (31).

### Mixed Viscosity

Mixed viscosity is measured by Brookfield viscometers at 12 rpm using spindle#2 at 25°C. The measurement is run three times and the average is calculated.

### Low-shear viscosity

The low-shear viscosity is measured via a TA Instruments AR2000 Rheometer having the following geometry:
40mm 2° stainless steel cone
40mm stainless steel plate
Standard Size DIN or Conical Concentric Cylinders

Using the data analysis program of the TA Instruments AR2000 Rheometer, collected data is then graphed and a point at the beginning of the run is recorded as the low-shear viscosity. Data should be run at least twice to ensure correlation of the recorded data.

### Foam Specific Volume

Foam specific volume is measure by placing a container of a known volume or marked with known volumes onto a mass balance, tarring the mass of the container and then dispensing from a foaming dispenser into the container. Record the resulting volume and the resulting mass of the foam. Dividing the volume by the mass of the foam results in the foam specific volume having the units of mL/g.

### Formulations

### Developer Formulations

**Table 1**

| Developer Composition 1 | | |
|---|---|---|
| Ingredient | Wt% (by weight Developer) | A Wt% (by weight Developer) |
| Hydrogen peroxide (50% active) | 10-15 | 12.3 |
| Phosphoric acid | 0.005 | 0.005 |
| Water | To 100 | To 100 |

**Table 2**

| Developer Composition 2 | | |
|---|---|---|
| Ingredient | Wt% (by weight of developer) | B Wt% (by weight of developer) |
| Etidronic acid | 0.02 | 0.02 |
| Hydrogen peroxide (50% active) | 1-20 | 18.3 |
| Water | To 100 | To 100 |

**Table 3**

| | Black shades | Black shades | red/auburn shades | red/auburn shades | light shades (blond shades) | light shades (blond shades) | Natural light brown | Dark natural brown |
|---|---|---|---|---|---|---|---|---|
| INCI Ingredient Name | Wt% (by weight of tint) | C Wt% (by weight of tint) | Wt% (by weight of tint) | D Wt% (by weight of tint) | Wt% (by weight of tint) | E Wt% (by weight of tint) | F Wt% (by weight of tint) | G Wt% (by weight of tint) |
| Ethoxydiglycol | 10-20 | 14.0 | 10-20 | 14.0 | 10-20 | 14.0 | -- | -- |
| Propylene Glycol | 1-5 | 4.0 | 1-5 | 4.0 | 1-5 | 4.0 | -- | -- |
| Isopropyl Alcohol | 1-10 | 5.0 | 1-10 | 5.0 | 1-10 | 5.0 | 5.0 | 5.0 |
| Soytrimonium Chloride | 1-10 | 7.3 | 1-10 | 7.3 | 1-10 | 7.3 | -- | -- |
| Oleth-5 | 1-10 | 3.0 | 1-10 | 3.0 | 1-10 | 3.0 | -- | -- |
| Oleic Acid | 1-10 | 3.25 | 1-10 | 3.75 | 1-10 | 3.75 | 4.0 | 4.5 |
| Oleth-2 | 0.5-5 | 1.5 | 0.5-5 | 1.5 | 0.1-5 | 1.5 | -- | -- |
| Ammonium hydroxide | 1-10 | 4.1 | 5-15 | 7.8 | 1-15 | 8.5 | 7.0 | 7.6 |
| Fragrance or Parfum | 0.5-5 | 1.2 | 0.5-5 | 1.2 | 0.1-5 | 1.2 | 1.2 | 1.2 |
| Cocamidopropyl Betaine | 5-15 | 6.8 | 1-15 | 6.8 | 1-10 | 6.8 | 3.3 | 3.3 |
| Trisodium Ethylenediamine Disuccinate | 1-15 | 6.7 | 1-10 | 6.7 | 1-10 | 6.7 | 6.7 | 6.7 |
| C11-15 Pareth-9 | 0.5-5 | 1.25 | 0.5-5 | 1.25 | 0.1-5 | 1.25 | 0.25 | 0.25 |
| C12-15 PARETH -3 | 0.05-3 | 0.5 | 0.1-2 | 0.5 | 0.05-1 | 0.5 | -- | -- |
| Citric Acid | 0.05-3 | 0.4 | 0.1-2 | 0.4000 | 0.05-1 | 0.4 | 0.4 | 0.2 |
| Erythorbic Acid | 0.05-3 | 0.4 | 0.1-2 | 0.4000 | 0.05-1 | 0.4 | 0.4 | 0.4 |
| Sodium Sulfite | 0.05-3 | 0.1 | 0.05-1 | 0.1000 | 0.01-1 | 0.1 | 0.1 | 0.1 |
| EDTA | 0.01-1 | 0.05 | 0.01-1 | 0.0500 | 0.01-1 | 0.05 | 0.05 | 0.05 |
| | | | | | | | | |
| P-Phenylenediamine | 0.5-5 | 1.98 | 0.05-1 | 0.15 | 0.01-1 | 0.08 | 0.45 | -- |
| Resorcinol | 0.05-3 | 0.9 | 0.05-1 | 0.2 | 0.01-1 | 0.04 | 0.4 | 0.95 |
| Phenyl methylpyrazolone | 0.05-3 | 0.1 | 0.05-1 | 0.1000 | 0.01-1 | 0.07 | -- | 0.05 |
| M-Aminophenol | 0.5-5 | 1.3 | -- | -- | 0.005-0.1 | 0.013 | 0.014 | 0.033 |
| P-Aminophenol | -- | -- | 0.1-2 | 0.66 | 0.005-0.1 | 0.015 | -- | 0.002 |
| 1-Naphthol | -- | -- | 0.01-1 | 0.0640 | 0.005-0.1 | 0.0260 | 0.0075 | 0.085 |
| N,N-Bis(2-Hydroxyethyl)-P-Phenylenediamine Sulfate | 0.5-5 | 1.2 | 0.005-0.1 | 0.015 | -- | -- | 0.04 | 0.05 |
| 4-Amino-2-hydroxytoluene | -- | -- | 0.1-2 | 0.47 | -- | -- | 0.006 | -- |
| 2-Methylresorcinol | -- | -- | -- | -- | 0.05-1 | 0.1970 | -- | -- |
| 1-phenyl-3-methyl-5-pyrazole | -- | -- | -- | -- | -- | -- | 0.1 | -- |
| Toluene-2,5-diamine | -- | -- | -- | - | - | -- | -- | 2.9 |
| 4-amino-2-hydroxytoluene | -- | -- | -- | -- | -- | -- | -- | 0.05 |
| Water | To 100% | To 100% | To 100% | To 100% | To 100% | To 100% | To 100% | To 100% |

**Table 4**

| Batch Description | Developer Choice | Tint/ Developer Ratio (by weight ratio) | Mixed Viscosity Brook field (cps) | Foamer Choice | Foam Specific Volume (mL /g) | Average Dispe nsed (g/stroke) | Amount Dispen sed (mL/stroke) | Low shear viscosity (cps) |
|---|---|---|---|---|---|---|---|---|
| Tint 1 (formulation C) | Developer 1 (Formulation A) | 62:62 | 838 | Squeeze foamer* | 9.2 | 1.3 | 12.0 | 100,000 |
| Tint 2 (formulation D) | Developer 2 (Formulation B) | 62:62 | 688 | Squeeze foamer | 9.6 | 1.3 | 12.5 | 130,000 |
| Tint 3 (formulation E) | Developer 2 (Formulation B) | 62:62 | 513 | Squeeze foamer* | 9.0 | 1.4 | 12.7 | 70,000 |

| | | | | | | | | |
|---|---|---|---|---|---|---|---|---|
| *The squeeze foamer is selected to have a structure similar to that shown in Figure 1 with the following dimensions selected: | | | | | | | | |

1. air ingress orifice size: 0.7 mm diameter
2. liquid ingress orifice size:1 mm diameter
3. liquid ingress orifice number of orifices: 3
4. mesh system in fluid connection after mixing chamber egress: 2 contiguous meshes with 170 µm openings.
5. mesh in fluid connection before dispensing orifice: 70 µm opening
6. mixing chamber egress orifice size: 1 mm diameter
7. dip-tube diameter: 3 mm

**Table 5**

| Rheology Profile | | | | | |
|---|---|---|---|---|---|
| | | Shade | Low Shear 0.01 l/s (cps) | High Shear 500 l/s (cps) | Slope |
| Comparative | | | | | |
| Blaune | Original | 1 - medium blonde | 17 | 14 | -0.006 |
| Blaune | Original | 3NA - light brown | 17 | 12 | -0.010 |
| Blaune | Original | 4 - medium brown | 18 | 11 | -0.014 |
| Tint Composition | Developer Composition | | | | |
| Formulation F | Formulation B | dark natural brown | 40000 | 60 | -79.882 |
| Formulation G | Formulation B | natural light brown | 29000 | 70 | -57.861 |

Table 5 shows a commercially available hair colorant that is delivered as a foam in 3 different shades. The product is sold by Kao Corporation under the Awa Blaune product name in Japan.

**Table 6**

| Hair Colorant Compositions | | | | | | |
|---|---|---|---|---|---|---|
| Ingredient | 1 | 2 | 3 | 4 | 5 | 6 |
| Ammonium Carbonate | 12.0 | - | - | 10.0 | 6.0 | - |
| Ammonium Hydrogen Carbonate | - | 7.0 | 6.0 | - | - | 8.0 |
| Ammonium Carbamate | - | 7.0 | 4.0 | - | - | 8.0 |
| Potassium Hydrogen Carbonate | - | - | - | - | - | - |
| Sodium Glycinate | 4.0 | 6.0 | 5.0 | 6.0 | 4.0 | 5.0 |
| Crodafos® CES (Cetearyl alcohol, dicetyl phosphate & ceteth-10 phosphate) | 8.0 | 10.0 | 6.0 | 6.0 | 4.0 | 10.0 |
| Sodium Palmytoyl Sarcosinate | - | - | - | - | - | - |
| Sodium Carboxymethyl Lauryl Glucoside | - | - | - | - | - | - |
| Sodium Alkyl Glyceryl Sulphonate | - | - | - | - | - | - |
| Behentrimonium Chloride | - | - | - | - | - | - |
| Steareth-100 | 1.2 | - | - | 0.6 | - | - |
| Steareth-200 | - | 1.25 | - | - | 1.0 | 2 |
| Ceteareth-25 | - | - | 0.6 | - | - | - |
| Cetyl Alcohol | 2.0 | - | - | 1.0 | - | 2.0 |
| Stearyl Alcohol | 0.8 | - | - | 1.0 | - | 4.0 |
| Cetearyl alcohol | - | 3.75 | 1.4 | - | - | - |
| p-phenylene diamine | - | - | 1.2 | 0.2 | 1.6 | - |
| p-amino phenol | - | - | - | 0.8 | - | 0.6 |
| 2,5-diaminotoluene sulphate | - | - | 0.4 | - | - | 0.2 |
| m-aminophenol | - | - | 0.2 | - | 0.4 | - |
| Resorcinol | - | - | - | 0.8 | - | 1.0 |
| napthol | - | - | 0.4 | - | 0.06 | - |
| 4-amino-2-hydroxy toluene | - | - | - | 0.6 | - | 0.4 |
| Basic red 51 | - | - | 0.4 | - | - | - |
| Basic yellow 87 | - | - | 0.6 | - | - | - |
| Amidomethicone(DCAP 6087) | - | - | - | - | - | 0.5 |
| Polyquaternium-22 (Merquat 295) | - | - | - | 0.2 | | |
| Polyquaternium-37 & Mineral oil (Salcare SC95) | 0.25 | 0.5 | - | 0.4 | - | - |
| Xanthan gum | 0.4 | - | - | - | - | - |
| Acrylates Steareth-20 Methacrylate Copolymer (Aculyn ® 22) | - | - | 1.5 | - | - | - |
| EDTA (tetrasodium salt) | 0.2 | 0.2 | 0.2 | 0.2 | 0.2 | 0.2 |
| Sodium sulphite | 0.2 | 0.2 | 0.2 | 0.2 | 0.2 | 0.2 |
| Ascorbic Acid | 0.2 | 0.2 | 0.2 | 0.2 | 0.2 | 0.2 |
| Propylene Glycol | 3.0 | 3.0 | 3.0 | 3.0 | 3.0 | 3.0 |
| pH adjust to pH 9.0 | qs | qs | qs | qs | qs | qs |
| Water | qs | qs | qs | qs | qs | qs |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A personal care product comprising a manually-actuable, non-aerosol dispenser (25) equipped with a reservoir (3) comprising a reservoir volume, a mixing chamber (5) and a dispensing head, wherein the reservoir (3) contains a liquid hair coloring mixture comprising:
(i) an oxidative dye precursor,
(ii) a foaming agent,
(iii) an alkalizing agent
(iv) a thickening agent; and
(v) an oxidizing agent;
wherein the volume of the hair coloring mixture is less than the reservoir volume (27); wherein the mixed viscosity of the hair coloring mixture is about 0.4 Pascal seconds (400 cps) to about 1.5 Pascal seconds (1500 cps); wherein when the manually-actuable, non-aerosol dispenser (25) is actuated the hair coloring mixture is mixed with air in an liquid to air ratio of from about 1:6 to about 1:15, preferably from 1:8 to 1:12, more preferably from 1:12 to 1:10, and is dispensed as a foam from the manually-actuable, non-aerosol dispenser (25);
wherein the reservoir (3) is fluidly connected to the mixing chamber (5), the mixing chamber (5) is fluidly connected to the dispensing head; wherein when the manually-actuable, nonaerosol dispenser (25) is actuated, a hair colorant product is dispensed as a foam;
wherein the mixing chamber (5) comprises at least one liquid ingress orifice (11), at least one air ingress orifice (9) and at least one mixing chamber egress orifice (13):
wherein the total area of the at least one air ingress orifice (9) is from about 0.62mm² to about 3.14mm², preferably from about 1.26mm² to about 1.88mm²;
wherein the total area of the at least one liquid ingress orifice is from about 1.5mm² to about 3mm², preferably from about 1.8mm² to about 2.3mm².

2. The personal care product of Claim 1 wherein the mixed viscosity of the hair coloring mixture is from 0.45 Pascal seconds (450 cps) to about 1.0 Pascal seconds (1000 cps).

3. The personal care product of any of the preceding claims wherein the hair coloring mixture has a low-shear viscosity of from 1.0 Pascal seconds (1000 cps) to about 150.0 Pascal seconds (150000 cps), preferably from 50.0 Pascal seconds (50000cps) to 150.0 Pascal seconds (150000cps), more preferably from 60.0 Pascal seconds to 140.0 Pascal seconds (60000 to 140000 cps).

4. The personal care product of any of the preceding claims wherein the thickening agent is selected from the group consisting of:
(a) salts of fatty acids represented by the formula: 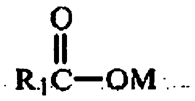 wherein R₁ is a hydrocarbon radical of the formula CₙH₂₋₁, CₙH₂ₙ₋₁ or CₙH₂ₙ₋₃ and n is an integer from 12 to 24 and mixtures thereof; M is hydrogen, sodium, potassium or ammonium
(b) xanthan gum, gellan gum, pectine, alginate, arabinogalctan, carageenan, rhamsan gum, furcellaran gum and mixtures thereof;
(c) hydroxyethylcellulose, carboxymethylcellulose, hydroxypropyltrimethyl ammonium guar gum and mixtures thereof;
(d) polyvinyl pyrrolidone, copolymers of vinylpyrrolidone, dimethylaminoethylmethacrylate and quaternary versions of the same with methyl sulfates, and polymers and copolymers of vinyl alcohol and vinyl acetate, polyacrylic acid, polyacrylamide, copolymers with esters of acrylic acid and methacrylic acid, copolymers of methylvinylether and maleic anhydride and mixtures thereof.

5. The personal care product of any of the preceding claims wherein the dispenser (25) further comprises a dip tube (7) in the reservoir (3) and connected to the ingress orifice (11) of the mixing chamber (5), wherein the dip tube (7) has a diameter of greater than 2.0 mm, preferably from about 2.0 mm to about 4.0 mm, more preferably from about 2.5 mm to about 3.5 mm.

6. The personal care product of any of the preceding claims wherein the hair coloring mixture is dispensed as a foam; wherein the foam has a foam specific volume from about 6 ml/g to 14 mUg, preferably from about 7.5 ml/g to about 12 mUg, more preferably from about 8 ml/g to 11 ml/g, still more preferably of about 10 mUg.

7. The personal care product of any of the preceding claims wherein the foaming agent is nonionic, anionic or amphoteric surfactant.

8. The personal care product of any preceding claims, wherein the dispenser (25) has a foam output per stroke or squeeze from about 0.5 gram/stroke to about 5.0 gram/stroke, preferably about 0.8 gram/stroke to 4.0 gram/stroke, more preferably from about 1.0 gram/stroke to about 4.0 gram/stroke, still more preferably from about 1.8 gram/stroke to about 2.2 gram/stroke.

9. The personal care product of any preceding claims, wherein the dispenser (25) has a foam output per stroke or squeeze from about 3 ml/stroke to about 70 ml/stroke, preferably about 8 ml/stroke to about 44 ml/stroke, more preferably from about 18 ml/stroke to about 22 ml/stroke.

10. The personal care product of any preceding claims, wherein the minimum time for the foam to retain its volume is for at least 10 seconds, preferably for at least 12 seconds, more preferably for at least 15 seconds.

11. The personal care product of any preceding claims, wherein the collapse of the foam is from 3 to 10 minutes.

12. The personal care product of any preceding claims, wherein the total area of the at least one mixing chamber egress orifice (13) is from about 0.314 mm² to about 9.42 mm², preferably from about 2.512 mm² to about 5.652 mm².

13. The personal care product of any preceding claims, wherein a diffuser plate (29) comprises the mixing chamber egress orifice (13).

14. Use of the personal care product of any preceding claims for coloring hair.

## Patentansprüche

1. Körperpflegeprodukt, das einen manuell betätigbaren aerosolfreien Spender (25) umfasst, der mit einem Reservoir (3) ausgerüstet ist, das ein Reservoirvolumen, eine Mischkammer (5) und einen Abgabekopf umfasst, wobei das Reservoir (3) eine flüssige Haarfärbemischung enthält, die Folgendes umfasst:
(I) eine oxidative Farbstoff-Präkursorsubstanz,
(II) einen Schaumbildner,
(III) ein Alkalisierungsmittel,
(IV) ein Verdickungsmittel und
(V) ein Oxidationsmittel,
wobei das Volumen der Haarfärbemischung geringer ist als das Reservoirvolumen (27), wobei die gemischte Viskosität der Haarfärbemischung etwa 0,4 Pascalsekunden (400 cps) bis etwa 1,5 Pascalsekunden (1500 cps) beträgt, wobei, wenn der manuell betätigbare aerosolfreie Spender (25) betätigt wird, die Haarfärbemischung mit Luft in einem Flüssigkeits-Luft-Verhältnis von etwa 1:6 bis etwa 1:15, vorzugsweise von 1:8 bis 1:12, mehr bevorzugt von 1:12 bis 1:10, gemischt wird und als ein Schaum von dem manuell betätigbaren aerosolfreien Spender (25) abgegeben wird,
wobei das Reservoir (3) in Fluidverbindung mit der Mischkammer (5) steht und die Mischkammer (5) in Fluidverbindung mit dem Abgabekopf steht, wobei, wenn der manuell betätigbare aerosolfreie Spender (25) betätigt wird, ein Haarfärbeprodukt als ein Schaum abgegeben wird,
wobei die Mischkammer (5) wenigstens eine Flüssigkeitseintrittsöffnung (11), wenigstens eine Lufteintrittsöffnung (9) und wenigstens eine Mischkammer-Austritts-öffnung (13) umfasst,
wobei die Gesamtfläche der wenigstens einen Lufteintrittsöffnung (9) etwa 0,62 mm² bis etwa 3,14 mm², vorzugsweise etwa 1,26 mm² bis etwa 1,88 mm², beträgt,
wobei die Gesamtfläche der wenigstens einen Flüssigkeitseintrittsöffnung etwa 1,5 mm² bis etwa 3 mm², vorzugsweise etwa 1,8 mm² bis etwa 2,3 mm², beträgt.

2. Körperpflegeprodukt nach Anspruch 1, wobei die gemischte Viskosität der Haarfärbemischung etwa 0,45 Pascalsekunden (450 cps) bis etwa 1,0 Pascalsekunden (1000 cps) beträgt.

3. Körperpflegeprodukt nach einem der vorstehenden Ansprüche, wobei die Haarfärbemischung eine niedrige Schemiskosität von 1,0 Pascalsekunden (1000 cps) bis etwa 150,0 Pascalsekunden (150000 cps), vorzugsweise von 50,0 Pascalsekunden (50000 cps) bis 150,0 Pascalsekunden (150000 cps), mehr bevorzugt von 60,0 Pascalsekunden bis 140,0 Pascalsekunden (60000 bis 140000 cps), aufweist.

4. Körperpflegeprodukt nach einem der vorstehenden Anspruche, wobei das Verdickungsmittel aus der Gruppe ausgewählt ist, die aus Folgendem besteht:
(a) Salzen von Fettsäuren, die durch die folgende Formel dargestellt sind: 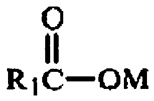 wobei R₁ ein Kohlenwasserstoffrest der Formel CₙH₂ₙ₋₁, CₙH₂ₙ₋₁ oder CₙH₂ₙ₋₃ ist und n eine ganze Zahl von 12 bis 24 ist, und Mischungen davon, M Wasserstoff, Natrium, Kalium oder Ammonium ist,
(b) Xanthangummi, Gellangummi, Pektin, Alginat, Arabinogalactan, Carrageen, Rhamsangiumni, Furcellarangiummi und Mischungen davon,
(c) Hydroxyethylcellulose, Carboxymethylcellulose, Hydroxypropyltrimethylammonium-Guargummi und Mischungen davon,
(d) Polyvinylpyrrolidon, Copolymeren von Vinylpyrrolidon, Dimethylaminoethylmethacrylat und quartären Versionen davon mit Methylsulfaten, und Polymeren und Copolymeren von Vinylalkohol und Vinylacetat, Polyacrylsäure, Polyacrylamid, Copolymeren mit Estern von Acrylsäure und Methacrylsäure, Copolymeren von Methylvinylether und Maleinsäureanhydrid und Mischungen davon.

5. Körperpflegeprodukt nach einem der vorstehenden Ansprüche, wobei der Spender (25) zudem ein Steigrohr (7) in dem Reservoir (3) umfasst und mit der Eintrittsöffnung (11) der Mischkammer (5) verbunden ist, wobei das Steigrohr (7) einen Durchmesser von größer als 2,0 mm, vorzugsweise von etwa 2,0 mm bis etwa 4,0 mm, mehr bevorzugt von etwa 2,5 mm bis etwa 3,5 mm, aufweist.

6. Körperpflegeprodukt nach einem der vorstehenden Ansprüche, wobei die Haarfärbemischung als ein Schaum abgegeben wird, wobei der Schaum ein spezifisches Schaumvolumen von etwa 6 ml/g bis 14 ml/g, vorzugsweise von etwa 7,5 ml/g bis etwa 12 ml/g, mehr bevorzugt von etwa 8 ml/g bis 11 ml/g, noch mehr bevorzugt von etwa 10 ml/g, aufweist.

7. Körperpflegeprodukt nach einem der vorstehenden Ansprüche, wobei der Schaumbildner ein nichtionisches, anionisches oder amphoteres Tensid ist.

8. Körperpflegeprodukt nach einem der vorstehenden Ansprüche, wobei der Spender (25) eine Schaumausbeute pro Stoß oder Zusammendrücken von etwa 0,5 Granum/Hub bis etwa 5,0 Gramm/Hub, vorzugsweise etwa 0,8 Gramm/Hub bis 4,0 Granum/Hub, mehr bevorzugt von etwa 1,0 Gramm/Hub bis etwa 4,0 Granum/Hub, noch mehr bevorzugt von etwa 1,8 Gramm/Hub bis etwa 2,2 Gramm/Hub, aufweist.

9. Körperpflegeprodukt nach einem der vorstehenden Ansprüche, wobei der Spender (25) eine Schaumausbeute pro Hub oder Zusammendrücken von etwa 3 ml/Hub bis etwa 70 ml/Hub, vorzugsweise etwa 8 ml/Hub bis etwa 44 ml/Hub, mehr bevorzugt von etwa 18 ml/Hub bis etwa 22 ml/Hub, aufweist.

10. Körperpflegeprodukt nach einem der vorstehenden Ansprüche, wobei die Mindestzeit für das Halten seines Volumens durch den Schaum mindestens 10 Sekunden, vorzugsweise mindestens 12 Sekunden, mehr bevorzugt mindestens 15 Sekunden, beträgt.

11. Körperpflegeprodukt nach einem der vorstehenden Ansprüche, wobei der Zerfall des Schaums in 3 bis 10 Minuten stattfindet.

12. Körperpflegeprodukt nach einem der vorstehenden Ansprüche, wobei die Gesamtfläche der wenigstens einen Mischkammer-Austrittsöffnung (13) etwa 0,314 mm² bis etwa 9,42 mm², vorzugsweise etwa 2,512 mm² bis etwa 5,652 mm², beträgt.

13. Körperpflegeprodukt nach einem der vorstehenden Ansprüche, wobei eine Zerstäuberplatte (29) die Mischkammer-Austrittsöffnung (13) umfasst.

14. Verwendung eines Körperpflegeprodukts nach einem der vorstehenden Ansprüche zum Färben von Haaren.

## Revendications

1. Produit de soins d'hygiène personnelle comprenant un distributeur non aérosol actionnable manuellement (25) équipé d'un réservoir (3) comprenant un volume de réservoir, une chambre de mélange (5) et une tête de distribution, dans lequel le réservoir (3) contient un mélange de coloration capillaire liquide comprenant :
(i) un précurseur de teinture oxydative,
(ii) un agent auto-moussant,
(iii) un agent alcalifiant
(iv) un agent épaississant ; et
(v) un agent oxydant ;
dans lequel le volume du mélange de coloration capillaire est inférieur au volume de réservoir (27) ; dans lequel la viscosité mélangée du mélange de coloration capillaire va d'environ 0,4 Pascal seconde (400 cP) à environ 1,5 Pascal seconde (1500 cP) ; dans lequel, lorsque le distributeur non aérosol actionnable manuellement (25) est actionné, le mélange de coloration capillaire est mélangé à de l'air dans un rapport de liquide sur air allant d'environ 1:6 à environ 1:15, de préférence de 1:8 à 1:12, plus préférablement de 1:12 à 1:10, et est distribué en tant que mousse à partir du distributeur non aérosol actionnable manuellement (25) ;
dans lequel le réservoir (3) est en communication fluidique avec la chambre de mélange (5), la chambre de mélange (5) est en communication fluidique avec la tête de distribution ; dans lequel, lorsque le distributeur non aérosol actionnable manuellement (25) est actionné, un produit de teinture capillaire est distribué en tant que mousse ;
dans lequel la chambre de mélange (5) comprend au moins une ouverture d'entrée de liquide (11), au moins un orifice d'entrée d'air (9) et au moins un orifice de sortie de chambre de mélange (13) :
dans lequel l'aire totale de l'au moins un orifice d'entrée d'air (9) va d'environ 0,62 mm² à environ 3,14 mm², de préférence d'environ 1,26 mm² à environ 1,88 mm² ;
dans lequel l'aire totale de l'au moins un orifice d'entrée de liquide va d'environ 1,5 mm² à environ 3 mm², de préférence d'environ 1,8 mm² à environ 2,3 mm².

2. Produit de soins d'hygiène personnelle selon la revendication 1, dans lequel la viscosité mélangée du mélange de coloration capillaire va de 0,45 Pascal seconde (450 cP) à environ 1,0 Pascal seconde (1000 cP).

3. Produit de soins d'hygiène personnelle selon l'une quelconque des revendications précédentes, dans lequel le mélange de coloration capillaire a une viscosité sous faible cisaillement allant de 1,0 Pascal seconde (1000 cP) à environ 150,0 Pascal secondes (150 000 cP), de préférence de 50,0 Pascal secondes (50 000 cP) à 150,0 Pascal secondes (150 000 cP), plus préférablement de 60,0 Pascal secondes à 140,0 Pascal secondes (60 000 à 140 000 cP).

4. Produit de soins d'hygiène personnelle selon l'une quelconque des revendications précédentes, dans lequel l'agent épaississant est choisi dans le groupe constitué de :
(a) sels d'acides gras représentés par la formule : 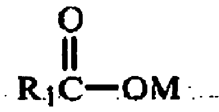 dans laquelle R₁ est un radical hydrocarbure de formule CₙH₂ₙ₋₁, CₙH₂ₙ₋₁ ou CₙH₂ₙ₋₃ et n est un nombre entier allant de 12 à 24 et leurs mélanges ; M est hydrogène, sodium, potassium ou ammonium
(b) gomme de xanthane, gomme gellane, pectine, alginate, arabinogalactane, carragahénine, gomme de rhamsane, gomme de furcellarane et leurs mélanges ;
(c) hydroxyéthylcellulose, carboxyméthylcellulose, gomme d'hydroxypropyltriméthyl-ammonium guar et leurs mélanges ;
(d) polyvinylpyrrolidone, copolymères de vinylpyrrolidone, méthacrylate de diméthylaminoéthyle et versions quaternaires de celui-ci avec des sulfates de méthyle, et polymères et copolymères d'alcool vinylique et d'acétate de vinyle, acide polyacrylique, polyacrylamide, copolymères avec des esters d'acide acrylique et d'acide méthacrylique, copolymères de méthylvinyléther et d'anhydride maléique et leurs mélanges.

5. Produit de soins d'hygiène personnelle selon l'une quelconque des revendications précédentes, dans lequel le distributeur (25) comprend en outre un tube immergé (7) dans le réservoir (3) et relié à l'orifice d'entrée (11) de la chambre de mélange (5), dans lequel le tube immergé (7) a un diamètre supérieur à 2,0 mm, de préférence d'environ 2,0 mm à environ 4,0 mm, plus préférablement d'environ 2,5 mm à environ 3,5 mm.

6. Produit de soins d'hygiène personnelle selon l'une quelconque des revendications précédentes, dans lequel le mélange de coloration capillaire est distribué en tant que mousse ; dans lequel la mousse a un volume spécifique de mousse d'environ 6 mL/g à 14 mL/g, de préférence d'environ 7,5 mL/g à environ 12 mL/g, plus préférablement d'environ 8 mL/g à 11 mL/g, encore plus préférablement d'environ 10 mL/g.

7. Produit de soins d'hygiène personnelle selon l'une quelconque des revendications précédentes, dans lequel l'agent moussant est un agent tensioactif non ionique, anionique ou amphotère.

8. Produit de soins d'hygiène personnelle selon l'une quelconque des revendications précédentes, dans lequel le distributeur (25) a une sortie de mousse par course ou pression d'environ 0,5 gramme/course à environ 5,0 grammes/course, de préférence environ 0,8 gramme/course à 4,0 grammes/course, plus préférablement d'environ 1,0 gramme/course à environ 4,0 grammes/course, encore plus préférablement d'environ 1,8 gramme/course à environ 2,2 grammes/course.

9. Produit de soins d'hygiène personnelle selon l'une quelconque des revendications précédentes, dans lequel le distributeur (25) a une sortie de mousse par course ou pression d'environ 3 mL/course à environ 70 mL/course, de préférence environ 8 mL/course à environ 44 mL/course, plus préférablement d'environ 18 mL/course à environ 22 mL/course.

10. Produit de soins d'hygiène personnelle selon l'une quelconque des revendications précédentes, dans lequel le temps minimum pendant lequel la mousse conserve son volume est pendant au moins 10 secondes, de préférence pendant au moins 12 secondes, plus préférablement pendant au moins 15 secondes.

11. Produit de soins d'hygiène personnelle selon l'une quelconque des revendications précédentes, dans lequel l'affaissement de la mousse va de 3 à 10 minutes.

12. Produit de soins d'hygiène personnelle selon l'une quelconque des revendications précédentes, dans lequel l'aire totale de l'au moins un orifice de sortie de chambre de mélange (13) va d'environ 0,314 mm² à environ 9,42 mm², de préférence d'environ 2,512 mm² à environ 5,652 mm².

13. Produit de soins d'hygiène personnelle selon l'une quelconque des revendications précédentes, dans lequel une plaque diffuseur (29) constitue l'orifice de sortie de chambre de mélange (13).

14. Utilisation du produit de soins d'hygiène personnelle selon l'une quelconque des revendications précédentes pour la coloration des cheveux.
